# FASCICULE DE BREVET EUROPEEN

(11) **EP 0 679 630 B1**
(45) Date de publication et mention de la délivrance du brevet: **06.05.1998**
(21) Numéro de dépôt: 95400701.9
(22) Date de dépôt: 29.03.1995
(51) Int. Cl.: C07C 65/26, A61K 31/19

(54) **Composés bicycliques-aromatiques, compositions pharmaceutiques et cosmétiques les contenant et utilisations**
Bicyclische aromatische Verbindungen, diese enthaltende pharmazeutische und kosmetische Zusammensetzungen und Verwendungen
Bicyclic aromatic compounds, pharmaceutical and cosmetic compositions containing them and uses

(30) Priorité: 26.04.1994 FR 9405019
(43) Date de publication de la demande: 02.11.1995
(73) Titulaire: CENTRE INTERNATIONAL DE RECHERCHES DERMATOLOGIQUES GALDERMA (C.I.R.D. GALDERMA), F-06560 Valbonne (FR)
(72) Inventeur: Bernardon, Jean-Michel, F-06650 Le Rouret (FR)
(74) Mandataire: Tezier Herman, Béatrice

(56) Documents cités:
- EP-A- 0 386 452
- Skin Pharmacol, vol.3, 1990, pages.256-267. Bailly et al.

## Description

La présente invention concerne, à titre de produits industriels nouveaux et utiles, des composés bicycliques-aromatiques. Elle concerne également l'utilisation de ces nouveaux composés dans des compositions pharmaceutiques destinées à un usage en médecine humaine ou vétérinaire, ou bien encore dans des compositions cosmétiques.

Il a déjà été décrit dans la demande de brevet EP 0386452 des composés bicycliques aromatiques différant dans leur structure par rapport aux composés de la présente invention par un radical méthylène supplémentaire entre les deux cycles aromatiques.

Les composés selon l'invention ont une activité marquée dans les domaines de la différenciation et de la prolifération cellulaire, et trouvent des applications plus particulièrement dans le traitement topique et systémique des affections dermatologiques liées à un désordre de la kératinisation, des affections dermatologiques (ou autres) à composante inflammatoire et/ou immunoallergique, et des proliférations dermiques ou épidermiques qu'elles soient bénignes ou malignes. Ces composés peuvent en outre être utilisés dans le traitement des maladies de dégénérescence du tissu conjonctif, pour lutter contre le vieillissement de la peau, qu'il soit photoinduit ou chronologique, et traiter les troubles de la cicatrisation. Ils trouvent par ailleurs une application dans le domaine ophtalmologique, notamment dans le traitement des cornéopathies. D'une manière générale, ils peuvent enfin trouver une application dans le traitement de toute maladie qui est associée à une modification de l'expression des récepteurs appartenant à la superfamille des récepteurs des hormones stéroïdiennes et thyroïdiennes.

On peut également utiliser les composés selon l'invention dans des compositions cosmétiques pour l'hygiène corporelle et capillaire.

Les composés selon l'invention peuvent être représentés par la formule générale (I) suivante : 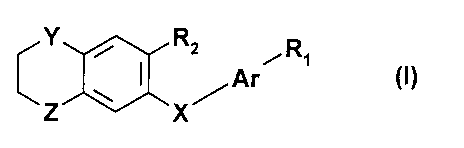 dans laquelle :
∗ R₁ représente un atome d'hydrogène, un radical -CH₃, un radical -CH₂-O-R₃, un radical -CH₂-O-CO-R₄, un radical -O-R₅, un radical -O-(CH₂)ₘ-(CO)ₙ-R₆, un radical -CO-R₇, un radical -CO-O-R₈, ou encore un radical S(O)ₚ-R₉, les valeurs m, n et p ainsi que les différents radicaux R₃ à R₉ ayant la signification donnée ci-après,
∗ R₂ représente un atome d'hydrogène ou un atome d'halogène, un radical alkyle inférieur de C₁-C₆, un radical -NO₂, un radical -O-COR₄, un radical -OR₉ ou bien encore un radical 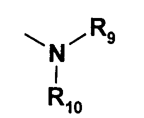 les radicaux R₄, R₉, et R₁₀ ayant la signification donnée ci-après,
* Ar représente un radical choisi parmi les radicaux de formules (a)-(e) suivantes : 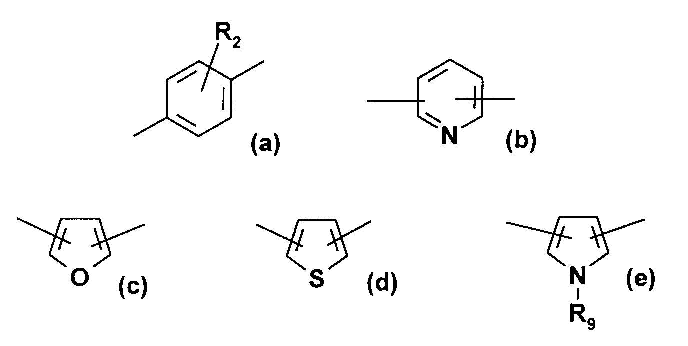 dans lesquelles R₂ a la signification ci-dessus, et R₉ celle donnée ci-après,
∗ X représente -O-, -S(O)ₜ- ou un radical -NR₉-, la valeur t et le radical R₉ ayant la signification donnée ci-après,
* Y et Z représentent -O-, -S(O)ₜ- ou encore un radical -CR₁₁R₁₂, la valeur t et les radicaux R₁₁ et R₁₂ ayant la signification donnée ci-après,
   étant entendu que dans tout ce qui précède :
   - m est un nombre entier égal à 1, 2 ou 3, n est un nombre entier égal à 0 ou 1, p est un nombre entier égal à 0, 1, 2 ou 3 et t est un nombre entier égal à 0, 1 ou 2,
   - R₃ représente un atome d'hydrogène ou un radical alkyle inférieur de C₁-C₆,
   - R₄ représente un radical alkyle inférieur de C₁-C₆,
   - R₅ représente un atome d'hydrogène ou un radical alkyle inférieur de C₁-C₆,
   - R₆ représente un radical alkyle inférieur de C₁-C₆ ou un hétérocycle,
   - R₇ représente un atome d'hydrogène, un radical alkyle inférieur de C₁-C₆ ou un radical : 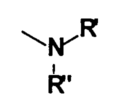 dans lequel R' et R'', identiques ou différents, représentent un atome d'hydrogène, un radical alkyle inférieur de C₁-C₆, un radical mono ou polyhydroxyalkyle, un radical aryle éventuellement substitué ou un reste d'aminoacide ou de peptide ou de sucre ou encore, pris ensemble, forment un hétérocycle,
   - R₈ représente un atome d'hydrogène, un radical alkyle linéaire ou ramifié ayant de 1 à 20 atomes de carbone, un radical alkényle, un radical mono ou polyhydroxyalkyle, un radical aryle ou aralkyle éventuellement substitué ou un reste de sucre ou un reste d'aminoacide ou de peptide,
   - R₉ représente un atome d'hydrogène ou un radical alkyle inférieur de C₁-C₆,
   - R₁₀ représente un atome d'hydrogène ou un radical alkyle inférieur de C₁-C₆,
   - R₁₁ représente un atome d'hydrogène ou un radical alkyle inférieur de C₁-C₆,
   - R₁₂ représente un atome d'hydrogène ou un radical alkyle inférieur de C₁-C₆,
   - Y et Z ne peuvent représenter en même temps un atome d'oxygène ou un radical -S(O)ₜ-.

L'invention vise également les sels des composés de formule (I) ci-dessus dans les cas où le radical R₁ représente une fonction acide carboxylique ou une fonction acide sulfonique ou porte une fonction amine, ou encore lorsque le radical R₂ représente une fonction amine, ainsi que les analogues chiraux desdits composés. Lorsque les composés selon l'invention se présentent sous forme de sels, il s'agit de préférence de sels d'un métal alcalin ou alcalino-terreux, ou encore de zinc ou d'une amine organique.

Selon la présente invention, on entend par radical alkyle inférieur un radical ayant de 1 à 6 atomes de carbone, de préférence les radicaux méthyle, éthyle, isopropyle, butyle, tertiobutyle et hexyle.

Par radical alkyle linéaire ou ramifié ayant de 1 à 20 atomes de carbone, on entend notamment les radicaux méthyle, éthyle, propyle, 2-éthyl-hexyle, octyle, dodécyle, hexadécyle et octadécyl.

Par radical monohydroxyalkyle, on entend un radical ayant de préférence 2 ou 3 atomes de carbone, notamment un radical 2-hydroxyéthyle, 2-hydroxypropyle ou 3-hydroxypropyle.

Par radical polyhydroxyalkyle on entend un radical contenant de préférence de 3 à 6 atomes de carbone et de 2 à 5 groupes hydroxyles tels que les radicaux 2,3-dihydroxypropyle, 2,3,4-trihydroxybutyle, 2,3,4,5-tétrahydroxypentyle ou le reste du pentaérythritol.

Par radical aryle, on entend de préférence un radical phényle éventuellement substitué par au moins un atome d'halogène, un hydroxyle ou une fonction nitro.

Par radical aralkyle, on entend de préférence le radical benzyle ou phénéthyle éventuellement substitué par au moins un atome d'halogène, un hydroxyle ou une fonction nitro.

Par radical alkényle, on entend un radical contenant de préférence de 2 à 5 atomes de carbone et présentant une ou plusieurs insaturations éthyléniques, tel que plus particulièrement le radical allyle.

Par reste de sucre, on entend un reste dérivant notamment de glucose, de galactose ou de mannose, ou bien encore de l'acide glucuronique.

Par reste d'aminoacide, on entend notamment un reste dérivant de la lysine, de la glycine ou de l'acide aspartique, et par reste de peptide on entend plus particulièrement un reste de dipeptide ou de tripeptide résultant de la combinaison d'acides aminés.

Par hétérocycle enfin, on entend de préférence un radical pipéridino, morpholino, pyrrolidino ou pipérazino, éventuellement substitué en position 4 par un radical alkyle en C₁-C₆ ou mono ou polyhydroxyalkyle tels que définis ci-dessus.

Lorsque le radical R₂ représente un atome d'halogène, celui-ci est de préférence un atome de fluor, de chlore ou de brome.

Parmi les composés de formule (I) ci-dessus rentrant dans le cadre de la présente invention, on peut notamment citer les suivants :
Acide 4-(5,6,7,8-tétrahydro-5,5,8,8-tétraméthyl-2-naphthyloxy)benzoïque
Acide 4-(5,6,7,8-tétrahydro-5,5,8,8-tétraméthyl-2-naphthylthio)benzoïque
Acide 4-(5,6,7,8-tétrahydro-5,5,8,8-tétraméthyl-2 naphthylsulfinyl) benzoïque
Acide 4-(5,6,7,8-tétrahydro-5,5,8,8-tétraméthyl-2-naphthylsulfonyl) benzoïque
Acide 4-(5,6,7,8-tétrahydro-5,5,8,8-tétraméthyl-2-naphthylamino)benzoïque
Acide 5-(5,6,7,8-tétrahydro-5,5,8,8-tétraméthyl-2-naphthylthio)-2-thiophene carboxylique
Acide 4-(3,5,5,8,8-pentaméthyl-5,6,7,8-tétrahydro-2-naphthyloxy) benzoïque
Acide 4-(3,5,5,8,8-pentaméthyl-5,6,7,8-tétrahydro-2-naphthylthio) benzoïque
Acide 4-(3-éthyl-5,5,8,8-tétramethyl-5,6,7,8-tétrahydro-2-naphthyloxy) benzoïque
Acide 4-(3-isopropyl-5,5,8,8-tétramethyl-5,6,7,8-tétrahydro-2-naphthyloxy) benzoïque
4-(5,6,7,8-tétrahydro-5,5,8,8-tétraméthyl-2-naphthyloxy)acétophenone
4-(5,6,7,8-tétrahydro-5,5,8,8-tétraméthyl-2-naphthyloxy)benzaldéhyde
Acide 4-(3-bromo-5,6,7,8-tétrahydro-5,5,8,8-tétramethyl-2-naphtyloxy) benzoïque
Acide 3-méthyl-4-(5,6,7,8-tétrahydro-5,5,8,8-tétramethyl-2-naphtylthio) benzoïque
Acide 3-méthyl-4-(3,5,5,8,8-pentaméthyl-5,6,7,8-tétrahydro-2-naphtylthio)benzoïque
Acide 3-méthyl-4-(3-éthyl-5,6,7,8-tétrahydro-5,5,8,8-tétramethyl-2-naphtyloxy) benzoïque
Acide 6-(3,5,5,8,8-pentaméthyl-5,6,7,8-tétrahydro-2-naphtylthio)nicotinique
Acide 2-hydroxy-4-(3,5,5,8,8-pentaméthyl-5,6,7,8-tétrahydrol-2-naphtylthio) benzoïque
Acide 2-chloro-4-(3,5,5,8,8-pentaméthyl-5,6,7,8-tétrahydro-2-naphtylthio) benzoïque
Acide 4-(3-éthyl-5,6,7,8-tétrahydro-5,5,8,8-tétramethyl-2-naphtylthio) benzoïque
Acide 4-(3-isopropyl-5,6,7,8-tétrahydro-5,5,8,8-tétramethyl-2-naphtylthio) benzoïque
Acide 4-(3-n-propyl-5,6,7,8-tétrahydro-5,5,8,8-tétramethyl-2-naphtylthio) benzoïque
4-(3,5,5,8,8-pentaméthyl-5,6,7,8-tétrahydro-2-naphtylthio)benzèneméthanol
4-(3,5,5,8,8-pentaméthyl-5,6,7,8-tétrahydro-2-naphtylthio)benzaldéhyde
N-éthyl-4-(3,5,5,8,8-pentaméthyl-5,6,7,8-tétrahydro-2-naphtylthio)benzamide
N-4-hydroxyphényl-4-(3,5,5,8,8-pentaméthyl-5,6,7,8-tétrahydro-2-naphtyl thio)benzamide.

Selon la présente invention, les composés de formule (I) plus particulièrement préférés sont ceux pour lesquels l'une au moins, et de préférence toutes, les conditions ci-dessous sont respectées :
- R₁ est un radical -CO-R₇
- R₂ est un radical alkyl inférieur ou un radical -OR₉
- Ar représente un radical de formule (a)
- et X représente -O-, -S- ou -NR₉-.

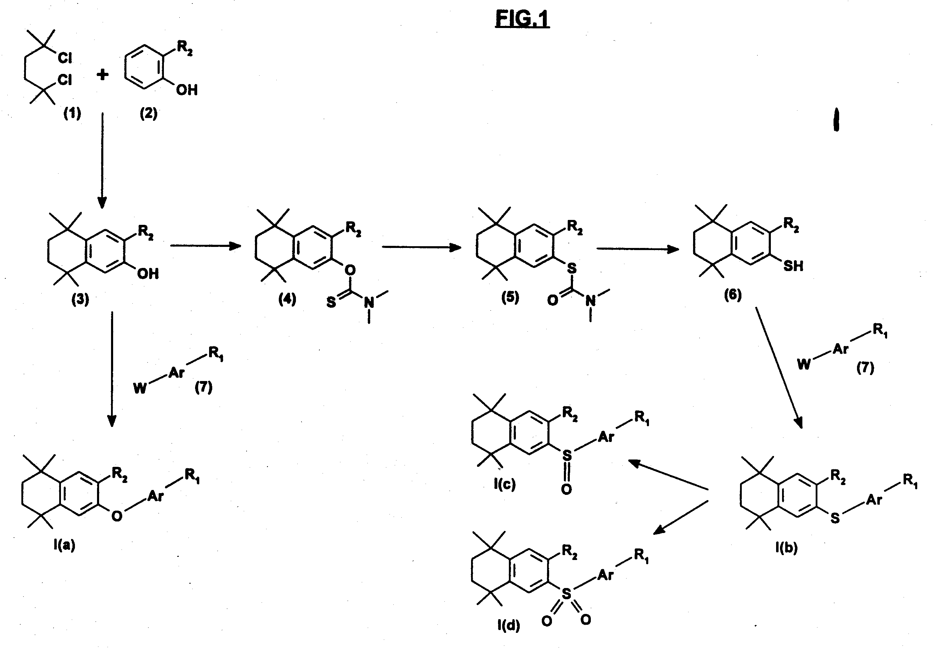

La présente invention a également pour objet les procédés de préparation des composés de formule (I), en particulier selon les schémas réactionnels donnés à la figure 1.

Ainsi, les composés de formule I(a) peuvent être obtenus (figure 1) à partir du sel de sodium du dérivé phénolique (3) par couplage avec un dérivé halogéné (7), de préférence un dérivé bromé ou iodé, en présence d'un complexe de bromure de cuivre et de diméthyl sulfure dans un solvant tel que la pyridine. Les dérivés phénoliques (3) peuvent, quant à eux, être obtenus par une réaction de type Friedel et Crafts à partir d'un phénol (2) et d'un dérivé dihalogéné (1) en présence d'un acide de Lewis par exemple le chlorure d'aluminium.

Les composés de formule I(b) peuvent être obtenus (figure 1) à partir du sel de sodium du dérivé thiol (6) par couplage avec un dérivé halogéné (7), de préférence un dérivé bromé ou iodé, en présence d'un catalyseur tel que certains complexes de métaux de transition dans un solvant alcoolique tel que l'alcool éthylique ou butylique. Comme catalyseurs convenables, on peut en particulier mentionner ceux dérivés du nickel ou du palladium par exemple les complexes de Ni ^{II} avec diverses phosphines et le tétrakis(triphénylphosphine)palladium(0). Les dérivés thiols (6) peuvent être obtenus à partir des dérivés phénoliques (3) via les dérivés dialkylthiocarbamates (4) et (5) selon les conditions générales décrites par M. Newman et H. Karnes dans J. Org. Chem 1966 31 3980-4.

Les dérivés de formule I(c) et I(d) peuvent être obtenus par oxydation du dérivé I(b), par exemple en employant l'acide méta chloroperbenzoïque.

Lorsque, dans la formule générale (I), X représente un radical -NR9, les composés peuvent alors être préparés suivant une réaction de type Ullman par déplacement nucléophilique direct d'un dérivé halogéné (7), de préférence iodé, par un dérivé aniline (8), en présence d'une base tel le carbonate de potassium ou la N-méthylmorpholine et de cuivre :

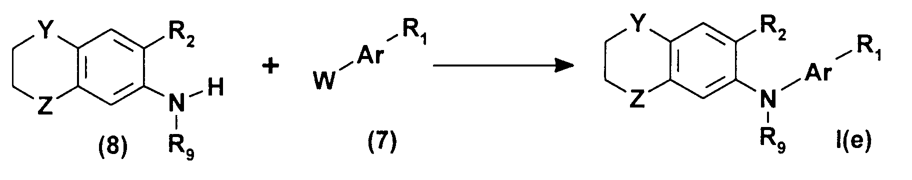

Dans les formules et réactions ci-dessus, R₁, R₂ ont les mêmes significations que celles données ci-avant pour la formule générale (I) ou en sont des dérivés convenablement protégés pour être compatibles avec les conditions de couplage. En particulier lorsqu'ils représentent le radical hydroxy, celui-ci est protégé de préférence sous forme de tert-butyldiméthylsilyloxy ou de méthoxyéthoxyméthoxy. La déprotection est alors effectuée soit en présence de fluorure de tétrabutylammonium ou d'iodure de triméthylsilane soit en milieu acide (par exemple acide chlorhydrique).

La présente invention a également pour objet à titre de médicament les composés de formule (I) telle que définie ci-dessus.

Ces composés présentent une activité vis à vis de l'expression de certains marqueurs de différenciation dans les kératinocytes humains in vitro (Anal. Biochem., 192, pp 232-236, 1991) et/ou présentent une bonne activité comédolytique dans le test Souris-Rhino (Skin Pharmacology, 4, pp 65-73, 1991).

Les composés selon l'invention conviennent particulièrement bien dans les domaines de traitement suivants :
1) pour traiter les affections dermatologiques liées à un désordre de la kératinisation portant sur la différenciation et sur la prolifération notamment pour traiter les acnées vulgaires, comédoniennes, polymorphes, rosacées, les acnées nodulokystiques, conglobata, les acnées séniles, les acnées secondaires telles que l'acnée solaire, médicamenteuse ou professionnelle,
2) pour traiter d'autres types de troubles de la kératinisation, notamment les ichtyoses, les états ichtyosiformes, la maladie de Darrier, les kératodermies palmoplantaires, les leucoplasies et les états leucoplasiformes, le lichen cutané ou muqueux (buccal),
3) pour traiter d'autres affections dermatologiques liées à un trouble de la kératinisation avec une composante inflammatoire et/ou immuno-allergique et notamment toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale ; les composés peuvent également être utilisés dans certaines affections inflammatoires ne présentant pas de trouble de la kératinisation,
4) pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires,
5) pour traiter d'autres désordres dermatologiques tels que les dermatoses bulleuses et les maladies du collagène,
6) pour traiter certains troubles ophtalmologiques, notamment les cornéopathies,
7) pour réparer ou lutter contre le vieillissement de la peau, qu'il soit photo-induit ou chronologique ou pour réduire les pigmentations et les kératoses actiniques, ou toutes pathologies associées au vieillissement chronologique ou actinique,
8) pour prévenir ou guérir les stigmates de l'atrophie épidermique et/ou dermique induite par les corticostéroïdes locaux ou systémiques, ou toute autre forme d'atrophie cutanée,
9) pour prévenir ou traiter les troubles de la cicatrisation ou pour prévenir ou pour réparer les vergetures,
10) pour lutter contre les troubles de la fonction sébacée tels que l'hyperséborrhée de l'acnée ou la séborrhée simple,
11) dans le traitement ou la prévention des états cancéreux ou précancéreux,
12) dans le traitement d'affections inflammatoires telles que l'arthrite,
13) dans le traitement de toute affection d'origine virale au niveau cutané ou général,
14) dans la prévention ou le traitement de l'alopécie,
15) dans le traitement d'affections dermatologiques ou générales à composante immunologique,
16) dans le traitement d'affections du système cardiovasculaire telles que l'artériosclérose,

Dans les domaines thérapeutiques mentionnés ci-dessus, les composés selon l'invention peuvent être avantageusement employés en combinaison avec d'autres composés à activité de type rétinoïde, avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques. Par vitamines D ou leurs dérivés, on entend par exemple les dérivés de la vitamine D₂ ou D₃ et en particulier la 1,25-dihydroxyvitamine D₃. Par anti-radicaux libres, on entend par exemple l'α-tocophérol, la Super Oxyde Dismutate, l'Ubiquinol ou certains chélatants de métaux. Par α-hydroxy ou α-céto acides ou leurs dérivés, on entend par exemple les acides lactique, malique, citrique, glycolique, mandélique, tartrique, glycérique ou ascorbique ou leurs sels, amides ou esters. Enfin, par bloqueurs de canaux ioniques, on entend par exemple le Minoxidil (2,4-diamino-6-pipéridino-pyrimidine-3-oxyde) et ses dérivés.

La présente invention a également pour objet des compositions médicamenteuses contenant au moins un composé de formule (I) telle que définie ci-dessus, l'un de ses analogues chiraux ou un de ses sels.

La présente invention a donc ainsi pour objet une nouvelle composition médicamenteuse destinée notamment au traitement des affections susmentionnées, et qui est caractérisée par le fait qu'elle comprend, dans un support pharmaceutiquement acceptable et compatible avec le mode d'administration retenu pour cette dernière, au moins un composé de formule (I), l'un de ses analogues chiraux ou un de ses sels.

L'administration des composés selon l'invention peut être effectuée par voie entérale, parentérale, topique ou oculaire.

Par voie entérale, les médicaments peuvent se présenter sous forme de comprimés, de gélules, de dragées, de sirops, de suspensions, de solutions, de poudres, de granulés, d'émulsions, de microsphères ou de nanosphères ou de vésicules lipidiques ou polymériques permettant une libération contrôlée. Par voie parentérale, les compositions peuvent se présenter sous forme de solutions ou de suspensions pour perfusion ou pour injection.

Les composés selon l'invention sont généralement administrés à une dose journalière d'environ 0,01 mg/kg à 100 mg/Kg en poids corporel, et ceci à raison de 1 à 3 prises.

Par voie topique, les compositions pharmaceutiques à base de composés selon l'invention sont plus particulièrement destinées au traitement de la peau et des muqueuses et peuvent alors se présenter sous forme d'onguents, de crêmes, de laits, de pommades, de poudres, de tampons imbibés, de solutions, de gels, de sprays, de lotions ou de suspensions. Elles peuvent également se présenter sous forme de microsphères ou nanosphères ou vésicules lipidiques ou polymériques ou de patches polymériques et d'hydrogels permettant une libération contrôlée. Ces compositions par voie topique peuvent par ailleurs se présenter soit sous forme anhydre, soit sous une forme aqueuse, selon l'indication clinique.

Par voie oculaire, ce sont principalement des collyres.

Ces compositions à usage topique ou oculaire contiennent au moins un composé de formule (I) telle que définie ci-dessus, ou l'un de ses isomères optiques ou géométriques ou encore l'un de ses sels, à une concentration de préférence comprise entre 0,001% et 5% en poids par rapport au poids total de la composition.

Les composés de formule (I) selon l'invention trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les aspects néfastes du soleil ou dans le traitement des peaux physiologiquement sèches, pour prévenir et/ou pour lutter contre le vieillissement photo-induit ou chronologique.

Dans le domaine cosmétique, les composés selon l'invention peuvent par ailleurs être avantageusement employés en combinaison avec d'autres composés à activité de type rétinoïde, avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques, tous ces différents produits étant tels que définis ci-avant.

La présente invention vise donc également une composition cosmétique qui est caractérisée par le fait qu'elle comprend, dans un support cosmétiquement acceptable et convenant à une application topique, au moins un composé de formule (I) telle que définie ci-dessus ou l'un de ses analogues chiraux ou l'un de ses sels, cette composition cosmétique pouvant notamment se présenter sous la forme d'une crème, d'un lait, d'une lotion, d'un gel, de microsphères ou nanosphères ou vésicules lipidiques ou polymériques, d'un savon ou d'un shampooing.

La concentration en composé de formule (I) dans les compositions cosmétiques selon l'invention est avantageusement comprise entre 0,001% et 3% en poids par rapport à l'ensemble de la composition.

Les compositions médicamenteuses et cosmétiques selon l'invention peuvent en outre contenir des additifs inertes ou même pharmacodynamiquement ou cosmétiquement actifs ou des combinaisons de ces additifs, et notamment : des agents mouillants; des agents dépigmentants tels que l'hydroquinone, l'acide azélaïque, l'acide caféïque ou l'acide kojique; des émollients; des agents hydratants comme le glycérol, le PEG 400, la thiamorpholinone, et ses dérivés ou bien encore l'urée; des agents antiséborrhéiques ou antiacnéiques, tels que la S-carboxyméthylcystéine, la S-benzyl-cystéamine, leurs sels ou leurs dérivés, ou le péroxyde de benzoyle; des antibiotiques comme l'érythromycine et ses esters, la néomycine, la clindamycine et ses esters, les tétracyclines; des agents antifongiques tels que le kétokonazole ou les polyméthylène-4,5 isothiazolidones-3; des agents favorisant la repousse des cheveux, comme le Minoxidil (2,4-diamino-6-pipéridino-pyrimidine-3-oxyde) et ses dérivés, le Diazoxide (7-chloro 3-méthyl 1,2,4-benzothiadiazine 1,1-dioxyde) et le Phénytoïn (5,4-diphényl-imidazolidine 2,4-dione); des agents anti-inflammatoires non stéroïdiens; des caroténoïdes et, notamment, le β-carotène; des agents anti-psoriatiques tels que l'anthraline et ses dérivés; et enfin les acides eicosa-5,8,11,14-tétraynoïque et eicosa-5,8,11-trynoïque, leurs esters et amides.

Les compositions selon l'invention peuvent également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des antioxydants, tels que l'α-tocophérol, le butylhydroxyanisole ou le butylhydroxytoluène.

On va maintenant donner, à titre d'illustration et sans aucun caractère limitatif, plusieurs exemples d'obtention de composés actifs de formule (I) selon l'invention, ainsi que diverses formulations concrètes à base de tels composés.

### EXEMPLE 1

### Acide 4-(5,6,7,8-tétrahydro-5,5,8,8-tétraméthyl-2-naphthyloxy)benzoïque

### (a) Méthyl 4-(5,6,7,8-tétrahydro-5,5,8,8-tétraméthyl-2-naphthyloxy)benzoate

Dans un tricol et sous courant d'azote, on introduit 5,3 g (15 mmoles) de 5,6,7,8-tétrahydro-5,5,8,8-tétraméthyl-2-naphthol et 70 ml de pyridine, et ajoute par petites quantités 430 mg (15 mmoles) d'hydrure de sodium (80% dans l'huile). On agite pendant trente minutes, on ajoute sucessivement 3,9 g (15 mmoles) de méthyl 4-iodobenzoate et 4,6 g (22,5 mmoles) d'un complex de bromure de cuivre et de diméthyl sulfure, et on chauffe à reflux pendant seize heures. On évapore à sec le milieu réactionnel, reprend par l'eau et l'éther éthylique, décante la phase organique, sèche sur sulfate de magnèsium, et évapore. Le résidu obtenu est purifié par chromatographie sur colonne de silice élué avec du dichlorométhane et de l'hexane (50-50). Après évaporation des solvants, on recueille 3,4 g (67%) de l'ester méthylique attendu.

### (b) Acide 4-(5,6,7,8-tétrahydro-5,5,8,8-tétraméthyl-2-naphthyloxy)benzoïque

Dans un ballon, on introduit 3,4 g (10 mmoles) de l'ester obtenu au point (a) précédent, 40 ml de THF et 40 ml d'une solution de soude méthanolique (2N), et on agite l'ensemble à température ambiante pendant huit heures. On évapore à sec le milieu réactionnel, reprend par l'eau, acidifie à pH 1, extrait avec de l'éther éthylique, décante la phase organique, sèche sur sulfate de magnèsium, et évapore. Le résidu obtenu est purifié par chromatographie sur colonne de silice élué avec un mélange de dichlorométhane et d'éther éthylique (97-3). On recueille, après évaporation des solvants, 2,3 g (71%) de l'acide attendu de point de fusion 234-5°C.

### EXEMPLE 2

### Acide 4-(5,6,7,8-tétrahydro-5,5,8,8-tétraméthyl-2-naphthylthio)benzoïque

### (a) éthyl 4-(5,6,7,8-tétrahydro-5,5,8,8-tétraméthyl-2-naphthylthio)benzoate

Dans un ballon, on introduit 30 ml d'alcool éthylique, puis on ajoute par petites quantités 500 mg (23 mmoles) de sodium et on agite l'ensemble pendant trente minutes. On ajoute ensuite sucessivement 2 g (9,1 mmoles) de 5,6,7,8-tétrahydro-5,5,8,8-tétraméthyl-2-naphthylthiol, 2,4 g (9,1 mmoles) de méthyl 4-iodobenzoate et 100 mg (0,09 mmole) de tétrakis(triphénylphosphine)-palladium (0), puis on chauffe le tout à reflux pendant quatre heures. On évapore le milieu réactionnel, reprend par l'eau et l'acétate d'éthyle, décante la phase organique, lave à l'eau, sèche sur sulfate de magnésium, et évapore. Le résidu obtenu est purifié par chromatographie sur colonne de silice élué avec du dichlorométhane. Après évaporation des solvants, on recueille 2,1 g (63%) d'ester éthylique.

### (b) acide 4-(5,6,7,8-tétrahydro-5,5,8,8-tétraméthyl-2-naphthylthio)benzoïque

De manière analogue à l'exemple 1(b) ci-dessus, à partir de 1,9 g (5,2 mmoles) de l'ester éthylique obtenu au point (a) précédent, on obtient 1,6 g (90%) de l'acide attendu de point de fusion 187-8°C.

### EXEMPLE 3

### Acide 4-(5,6,7,8-tétrahydro-5,5,8,8-tétraméthyl-2-naphthylsulfinyl)benzoïque

### (a) éthyl 4-(5,6,7,8-tétrahydro-5,5,8,8-tétraméthyl-2-naphthylsulfinyl)-benzoate

Dans un ballon, on introduit 1,1 g (3,1 mmoles) d' éthyl 4-(5,6,7,8-tétrahydro-5,5,8,8-tétraméthyl-2-naphthylthio)benzoate et 30 ml de dichlorométhane, et on ajoute ensuite 970 mg (3,1mmoles) d'acide méta-chloroperbenzoïque. On agite l'ensemble à température ambiante pendant deux heures, verse le milieu réactionnel dans l'eau, extrait avec du dichlorométhane, décante la phase organique, sèche sur sulfate de magnésium, et évapore. Le résidu est purifié par chromatographie sur colonne de silice élué avec du dichlorométhane. Après évaporation des solvants, on recueille 1 g (84%) de l'ester attendu.

### (b) acide 4-(5,6,7,8-tétrahydro-5,5,8,8-tétraméthyl-2-naphthylsulfinyl)-benzoïque

De manière analogue à l'exemple 1(b) ci-dessus, à partir de 960 mg (2,5 mmoles) de l'ester éthylique obtenu au point (a) précédent, on obtient 890 mg (99%) de l'acide attendu de point de fusion 214-6°C.

### EXEMPLE 4

### Acide 4-(5,6,7,8-tétrahydro-5,5,8,8-tétraméthyl-2-naphthylsulfonyl) benzoïque

### (a) éthyl 4-(5,6,7,8-tétrahydro-5,5,8,8-tétraméthyl-2-naphthylsulfonyl)-benzoate

Dans un ballon, on introduit 1,05 g (2,8 mmoles) d' éthyl 4-(5,6,7,8-tétrahydro-5,5,8,8-tétraméthyl-2-naphthylthio)benzoate et 30 ml de dichlorométhane, et on ajoute ensuite 2,45 g (7,12 mmoles) d'acide méta-chloroperbenzoïque. On agite l'ensemble à température ambiante pendant deux heures, verse le milieu réactionnel dans l'eau, extrait avec du dichlorométhane, décante la phase organique, sèche sur sulfate de magnésium, et évapore. Le résidu est purifié par chromatographie sur colonne de silice élué avec du dichlorométhane. Après évaporation des solvants, on recueille 1,09 g (95%) de l'ester attendu.

### (b) acide 4-(5,6,7,8-tétrahydro-5,5,8,8-tétraméthyl-2-naphthylsulfonyl)-benzoïque

De manière analogue à l'exemple 1(b) ci-dessus, à partir de 1,09 g (2,7 mmoles) de l'ester ethylique obtenu au point (a) précédent, on obtient 1 g (99%) de l'acide attendu de point de fusion 218-20°C..

### EXEMPLE 5

### Acide 4-(5,6,7,8-tétrahydro-5,5,8,8-tétraméthyl-2-naphthylamino)benzoïque

Dans un ballon, on introduit sucessivement 1,02 g (5 mmoles) de 5,6,7,8-tétrahydro-5,5,8,8-tétraméthyl-2-naphthylamine, 1,24 g (5 mmoles) d'acide 4-iodobenzoïque, 820µl (7,5 mmoles) de N-méthylmorpholine, 360 mg (2,5 mmoles) de Cu₂O et 15 ml de dioxanne. On chauffe l'ensemble à reflux pendant 24 heures, verse le milieu réactionnel dans 15 ml d'acide chlorhydrique 5N, filtre le précipité, et lave ce dernier à l'eau. Le solide est trituré dans l'alcool éthylique, filtré, séché. On recueille alors 200 mg (12%) de l'acide attendu de point de fusion 262-4°C.

### EXEMPLE 6

### Acide 5-(5,6,7,8-tétrahydro-5,5,8,8-tétraméthyl-2-naphthylthio)-2-thiophene carboxylique

Dans un ballon, on introduit 40 ml d'alcool n-butylique, puis on ajoute par petites quantités 300 mg (13 mmoles) de sodium, et on agite l'ensemble pendant trente minutes. On ajoute ensuite sucessivement 1,1 g (5 mmoles) de 5,6,7,8-tétrahydro-5,5,8,8-tétraméthyl-2-naphthylthiol, 1,1 g (5 mmoles) de méthyl 5-bromo-2-thiophenecarboxylate et 230 mg (0,2 mmole) de tétrakis(triphénylphosphine)palladium(0), puis on chauffe le tout à reflux pendant quatre heures. On évapore le milieu réactionnel, reprend par l'eau et l'acétate d'éthyle, décante la phase organique, lave à l'eau, sèche sur sulfate de magnésium, évapore. Le résidu obtenu est purifié par chromatographie sur colonne de silice élué avec de l'acétate d'éthyle et de l'hexane (60-40). Après évaporation des solvants, on recueille 180 mg (10%) d'acide 5-(5,6,7,8-tétrahydro-5,5,8,8-tétraméthyl-2-naphthylthio)-2-thiophènecarboxylique de point de fusion 141-2°C.

### EXEMPLE 7

### Acide 4-(3,5,5,8,8-pentaméthyl-5,6,7,8-tétrahydro-2-naphthyloxy)benzoïque

### (a) 3,5,5,8,8-pentaméthyl-5,6,7,8-tétrahydro-2-naphthol

Dans un tricol, on introduit 50,8 g (0,27 mole) de 2,5-dichloro-2,5-diméthylhexane, 30 g (0,27 mole) de 2-méthylphénol et 500 ml de dichlorométhane. A 0°C, on ajoute par petites quantités 14,8 g (0,11 mole) de chlorure d'aluminium et on agite le tout à température ambiante pendant douze heures. On verse le milieu réactionnel dans l'eau glacée, extrait avec le dichlorométhane, décante la phase organique, lave à l'eau bicarbonatée, sèche sur sulfate de magnésium, et évapore. Le résidu obtenu est trituré dans l'hexane, filtré, et on recueille alors, après sèchage, 54,4 g (90%) de phénol attendu de point de fusion 125-6°C.

### (b) méthyl 4-(3,5,5,8,8-pentaméthyl-5,6,7,8-tétrahydro-2-naphthyloxy)benzoate

De manière analogue à l'exemple 1(a) ci-dessus, par réaction de 1,1 g (5 mmoles) de 3,5,5,8,8-pentaméthyl-5,6,7,8-tétrahydro-2-naphthol avec 0,89 g (4,1 mmoles) de méthyl 4-bromobenzoate, on obtient 670 mg (46%) d'ester méthylique attendu de point de fusion 133-5°C.

### (c) acide 4-(3,5,5,8,8-pentaméthyl-5,6,7,8-tétrahydro-2-naphthyloxy)benzoïque

De manière analogue à l'exemple 1(b) ci-dessus, à partir de 670 mg (1,9 mmoles) de l'ester méthylique obtenu au point (b) précédent, on obtient 620 mg (96%) d'acide attendu de point de fusion 208-10°C.

### EXEMPLE 8

### Acide 4-(3,5,5,8,8-pentaméthyl-5,6,7,8-tétrahydro-2-naphthylthio)benzoïque

### (a) O-3,5,5,8,8-pentaméthyl-5,6,7,8-tétrahydro-2-naphthyldimethylthiocarbamate

Dans un ballon et sous courant d'azote, on introduit 4,1 g (0,138 mole) d'hydrure de sodium (80% dans l'huile) et 200 ml de DMF. On refroidit à 0°C et on ajoute goutte à goutte une solution de 25,2 g (0,115 mole) de 3,5,5,8,8-pentaméthyl-5,6,7,8-tétrahydro-2-naphthol dans 100 ml de DMF et on agite le tout jusqu'à cessation du dégagement gazeux. On ajoute ensuite une solution de 18,55 g (0,15 mole) de chlorure de dimethylthiocarbamoyle dans 200 ml de DMF et on agite à nouveau l'ensemble pendant huit heures à température ambiante. On verse alors le milieu réactionnel dans l'eau, extrait avec de l'acétate d'éthyle, décante la phase organique, lave à l'eau, sèche sur sulfate de magnésium, et enfin évapore. Le solide obtenu est purifié par chromatographie sur colonne de silice élué avec un mélange d'acétate d'éthyle et d'hexane (30-70). Après évaporation des solvants, on recueille 20 g (68%) du produit attendu de point de fusion 110-1°C.

### (b) S-3,5,5,8,8-pentaméthyl-5,6,7,8-tétrahydro-2-naphthyldimethylthiocarbamate

Dans un ballon et sous courant d'azote, on introduit 20,1 g (65,8 mmoles) du produit obtenu au point (a) précédent et on chauffe à 240°C pendant six heures. On extrait le milieu réactionnel avec du dichloromethane, lave à l'eau, décante la phase organique, sèche sur sulfate de magnésium, et évapore. On recueille 18,1 g (90%) du produit attendu de point de fusion 138-9°C.

### (c) 3,5,5,8,8-pentaméthyl-5,6,7,8-tétrahydro-2-naphthylthiol

Dans un ballon, on introduit 23 g (75 mmoles) du produit obtenu au point (b) précédent et 300 ml d'alcool methylique. On ajoute 30 g (75 mmoles) d'hydroxyde de sodium et on chauffe l'ensemble à reflux pendant trois heures. On évapore le milieu réactionnel, reprend par l'eau, acidifie avec de l'acide chlorhydrique concentré, et enfin on filtre. Le solide obtenu est lavé à l'eau, séché, et on recueille alors 18 g (99%) de 3,5,5,8,8-pentaméthyl-5,6,7,8-tétrahydro-2-naphthylthiol de point de fusion 97-8°C.

### (d) éthyl 4-(3,5,5,8,8-pentaméthyl-5,6,7,8-tétrahydro-2-naphthylthio)benzoïque

De manière analogue à l'exemple 2 (a) ci-dessus, par réaction de 2 g (8,5 mmoles) de 3,5,5,8,8-pentaméthyl-5,6,7,8-tétrahydro-2-naphthylthiol avec 2,24 g (8,5 mmoles) de méthyl 4-iodobenzoate, on obtient, après recristallisation dans l'alcool éthylique, 2 g (63%) d'ester ethylique de point de fusion 109-10°C.

### (e) Acide 4-(3,5,5,8,8-pentaméthyl-5,6,7,8-tétrahydro-2-naphthylthio)benzoïque

De manière analogue à l'exemple 1 (b) ci-dessus, à partir de 2 g (5,2 mmoles) de l'ester éthylique obtenu au point (d) précédent, on obtient 1,58 g (85%) de l'acide attendu de point de fusion 253-4°C.

### EXEMPLE 9

### Acide 4-(3-ethyl-5,5,8,8-tétraméthyl-5,6,7,8-tétrahydro-2-naphthyloxy) benzoïque

### (a) 3-éthyl-5,5,8,8-tétraméthyl-5,6,7,8-tétrahydro-2-naphthol

De manière analogue à l'exemple 7 (a) ci-dessus, par réaction de 15 g (0,123 mole) de 2-éthylphenol avec 22,5 g (0,123 mole) de 2,5-dichloro-2,5-diméthylhexane, on obtient, après chromatographie sur colonne de silice élué avec du dichlorométhane, 25,4 g (89%) du phenol attendu de point de fusion 88-9°C.

### (b) méthyl 4-(3-éthyl-5,5,8,8-tétraméthyl-5,6,7,8-tétrahydro-2-naphthyloxy) benzoate

De manière analogue à l'exemple 1(a) ci-dessus, par réaction de 2 g (8,6 mmoles) de 3-éthyl-5,5,8,8-tétraméthyl-5,6,7,8-tétrahydro-2-naphthol avec 1,52 g (7 mmoles) de méthyl 4-bromobenzoate, on obtient 1,5 g (56%) d'ester méthylique attendu sous forme d'une huile jaune.

### (c) acide 4-(3-éthyl-5,5,8,8-tétraméthyl-5,6,7,8-tétrahydro-2-naphthyloxy) benzoïque

De manière analogue à l'exemple 1(b) ci-dessus, à partir de 1,5 g (4 mmoles) de l'ester méthylique obtenu au point (b) précédent, on obtient 1,24 g (86%) d'acide attendu de point de fusion 195-6°C.

### EXEMPLE 10

### Acide 4-(3-isopropyl-5,5,8,8-tétraméthyl-5,6,7,8-tétrahydro-2-naphthyloxy) benzoïque

### (a) 3-isopropyl-5,5,8,8-tétraméthyl-5,6,7,8-tétrahydro-2-naphthol

De manière analogue à l'exemple 7 (a) ci-dessus, par réaction de 30 g (0,22 mole) de 2-isopropylphenol avec 40,3 g (0,22 mole) de 2,5-dichloro-2,5-diméthylhexane, et après chromatographie sur colonne de silice élué avec un mélange de dichlorométhane et d'hexane (40-60), on obtient 48,9 g (90%) du phenol attendu de point de fusion 79-80°C.

### (b) méthyl 4-(3-isopropyl-5,5,8,8-tétraméthyl-5,6,7,8-tétrahydro-2-naphthyloxy) benzoate

De manière analogue à l'exemple 1(a) ci-dessus, par réaction de 2 g (8,1 mmoles) de 3-isopropyl-5,5,8,8-tétraméthyl-5,6,7,8-tétrahydro-2-naphthol avec 1,45 g (6,7 mmoles) de méthyl 4-bromobenzoate, on obtient 840 mg (33%) d'ester méthylique attendu de point de fusion 115-6°C.

### (c) acide 4-(3-isopropyl-5,5,8,8-tétraméthyl-5,6,7,8-tétrahydro-2-naphthyloxy) benzoïque

De manière analogue à l'exemple 1(b) ci-dessus, à partir de 800 mg (2,1 mmoles) de l'ester méthylique obtenu au point (b) précédent, on obtient 690 mg (90%) d'acide attendu de point de fusion 205-6°C.

### EXEMPLE 11

### 4-(5,6,7,8-tétrahydro-5,5,8,8-tétraméthyl-2-naphthyloxy)acétophenone

De manière analogue à l'exemple 1(a) ci-dessus, par réaction de 6,6 g (32 mmoles) de 5,6,7,8-tétrahydro-5,5,8,8-tétraméthyl-2-naphthol avec 5,4 g (27 mmoles) de 4-bromoacetophenone, on obtient 6,3 g (72%) de la cétone attendue sous forme d'une huile légèrement jaune.

### EXEMPLE 12

### 4-(5,6,7,8-tétrahydro-5,5,8,8-tétraméthyl-2-naphthyloxy)benzaldéhyde

De manière analogue à l'exemple 1(a) ci-dessus, par réaction de 5,3 g (15 mmoles) de 5,6,7,8-tétrahydro-5,5,8,8-tétraméthyl-2-naphthol avec 3,1 g (16,5 mmoles) de 4-bromobenzaldéhyde, on obtient, après purification par chromatographie sur colonne de silice élué avec un mélange de dichlorométhane et d'hexane (50-50), 2,4 g de l'aldéhyde attendu de point de fusion 75-6°C.

### EXEMPLE 13

### Acide 4-(3-bromo-5,6,7,8-tétrahydro-5,5,8,8-tétramethyl-2-naphtyloxy) benzoïque.

### (a) 4-(3-bromo-5,6,7,8-tétrahydro-5,5,8,8-tétramethyl-2-naphtyloxy)benzoate de méthyle.

De manière anlogue à l'exemple 1(a) par réaction de 1 g (3,5 mmoles) de 3-bromo-5,6,7,8-tétrahydro-5,5,8,8-tétramethyl-2-naphtol avec 770 mg (2,94 mmoles) de 4-iodobenzoate de méthyle, on obtient 700 mg (58%) de l'ester méthylique attendu de point de fusion 135-6°C.

### (b) Acide 4-(3-bromo-5,6,7,8-tétrahydro-5,5,8,8-tétramethyl-2-naphtyloxy) benzoïque.

De manière analogue à l'exemple 1(b) à partir de 700 mg (1,6 mmole) de l'ester méthylique précédent, on obtient 650 mg (95%) d'acide 4-(3-bromo-5,6,7,8-tétrahydro-5,5,8,8-tétramethyl-2-naphtyloxy)benzoïque de point de fusion 229-30°C.

### EXEMPLE 14

### Acide 3-méthyl-4-(5,6,7,8-tétrahydro-5,5,8,8-tétramethyl-2-naphtylthio) benzoïque.

### (a) Acide 3-méthyl-4-iodobenzoïque.

Dans un tricol on introduit 20 g (0,132 mole) d'acide 3-méthyl-4-amino benzoïque 175 ml d'acide sulfurique (20%). A -10°C on ajoute goutte à goutte une solution de 11,9 g (0,172 mole) de nitrite de sodium dans 50 ml d'eau et agite pendant deux heures. Cette solution est introduite goutte à goutte par l'intermédiaire d'une ampoule réfrigérée à -5°C dans une solution de 35 g (0,211 mole) d'iodure de potassium 35,2 g (0,185 mole) d'iodure de cuivre et 175 ml d'acide sulfurique (20%). On agite pendant huit heures, filtre le milieu réactionnel, dissout le solide obtenu dans l'acétate d'éthyle, lave à l'eau puis avec une solution de sulfite de sodium,sèche sur sulfate de magnésium, évapore. On recueille 24,4 g (70%) d'acide 3-méthyl-4-iodobenzoïque de point de fusion 205-10°C.

### (b) 3-méthyl-4-iodobenzoate de méthyle.

Dans un ballon on introduit 24,4 g (0,093 mole) d'acide 3-méthyl-4-iodobenzoïque 250 ml de méthanol et ajoute goutte à goutte 2,5 ml d'acide sulfurique concentré. On chauffe à reflux pendant douze heures, évapore le milieu réactionnel, reprend avec acétate d'éthyle et eau, décante la phase organique, sèche sur sulfate de magnésium, évapore. Le résidu est trituré dans le méthanol, filtré, on recueille 21,9 g (85%) de l'ester méthylique attendu de point de fusion 58-9°C.

### (c) Acide 3-méthyl-4-(5,6,7,8-tétrahydro-5,5,8,8-tétramethyl-2-naphtylthio) benzoïque.

De manière analogue à l'exemple 2(a) par réaction de 2,4 g (11 mmoles) de 5,6,7,8-tétrahydro-5,5,8,8-tétramethyl-2-naphtylthiol avec 3 g (11 mmoles) de 3-méthyl-4-iodobenzoate de méthyle, on obtient directement 1,96 g (51%) d'acide 3-méthyl-4-(5,6,7,8-tétrahydro-5,5,8,8-tétramethyl-2-naphtylthio)benzoïque de point de fusion 195-6°C.

### EXEMPLE 15

### Acide 3-méthyl-4-(3,5,5,8,8-pentaméthyl-5,6,7,8-tétrahydro-2-naphtylthio) benzoïque.

### (a) 3-méthyl-4-(3,5,5,8,8-pentaméthyl-5,6,7,8-tétrahydro-2-naphtylthio)benzoate d'éthyle.

De manière analogue à l'exemple 2(a) par réaction de 2,55 g (11 mmoles) de 3,5,5,8,8-pentaméthyl-5,6,7,8-tétrahydro-2-naphtylthiol avec 3 g (11 mmoles) de 3-méthyl-4-iodobenzoate de méthyle, on obtient 1,86 g (43%) du produit attendu sous forme d'ester éthylique comme une huile orange.

### (b) acide 3-méthyl-4-(3,5,5,8,8-pentaméthyl-5,6,7,8-tétrahydro-2-naphtylthio) benzoïque.

De manière analogue à l'exemple 1(b) à partir de 1,86 g (4,7 mmoles) de l'ester éthylique précédent, on obtient 1,5 g (97%) d' acide 3-méthyl-4-(3,5,5,8,8-pentaméthyl-5,6,7,8-tétrahydro-2-naphtylthio)benzoïque de point de fusion 217-8°C.

### EXEMPLE 16

### Acide 3-méthyl-4-(3-éthyl-5,6,7,8-tétrahydro-5,5,8,8-tétramethyl-2-naphtyloxy) benzoïque.

### (a) 3-méthyl-4-(3-éthyl-5,6,7,8-tétrahydro-5,5,8,8-tétramethyl-2-naphtyloxy) benzoate de méthyle.

De manière anlogue à l'exemple 1(a) par réaction de 2,1 g (8,7 mmoles) de 3-éthyl-5,6,7,8-tétrahydro-5,5,8,8-tétramethyl-2-naphtol avec 2 g (7,2 mmoles) de 3-méthyl-4-iodo benzoate de méthyle, on obtient 2,18 g (79%) de l'ester méthylique attendu sous forme d'une huile jaune pâle.

### (c) acide 3-méthyl-4-(3-éthyl-5,6,7,8-tétrahydro-5,5,8,8-tétramethyl-2-naphtyloxy) benzoïque.

De manière analogue à l'exemple 1(b) à partir de 2,18 g (5,7 mmoles) de l'ester méthylique précédent, on obtient 1,9 g (90%) d'acide 3-méthyl-4-(3-éthyl-5,6,7,8-tétrahydro-5,5,8,8-tétramethyl-2-naphtyloxy)benzoïque de point de fusion 210-1°C.

### EXEMPLE 17

### Acide 6-(3,5,5,8,8-pentaméthyl-5,6,7,8,8-tétrahydro-2-naphtylthio)nicotinique.

### (a) 6-(3,5,5,8,8-pentaméthyl-5,6,7,8-tétrahydro-2-naphtylthio)nicotinate d'éthyle.

De manière analogue à l'exemple 2(a) par réaction de 800 mg (3,4 mmoles) de 3,5,5,8,8-pentaméthyl-5,6,7,8-tétrahydro-2-naphtylthiol avec 900 mg (3,4 mmoles) de 6-iodonicotinate de méthyle, on obtient 620 mg (47%) du produit attendu sous forme d'ester éthylique.

### (b) acide 6-(3,5,5,8,8-pentaméthyl-5,6,7,8-tétrahydro-2-naphtylthio)nicotinique.

De manière analogue à l'exemple 1(b) à partir de 620 mg (1,6 mmole) de l'ester éthylique précédent, on obtient 520 mg (90%) d' acide 6-(3,5,5,8,8-pentaméthyl-5,6,7,8-tétrahydro-2-naphtylthio)nicotinique de point de fusion 257-60°C.

### EXEMPLE 18

### Acide 2-hydroxy-4-(3,5,5,8,8-pentaméthyl-5,6,7,8-tétrahydro-2-naphtylthio) benzoïque.

### (a) 2-hydroxy-4-(3,5,5,8,8-pentaméthyl-5,6,7,8-tétrahydro-2-naphtylthio)benzoate d'éthyle.

De manière analogue à l'exemple 2(a) par réaction de 2 g (8,53 mmoles) de 3,5,5,8,8-pentaméthyl-5,6,7,8-tétrahydro-2-naphtylthiol avec 2,37 g (8,53 mmoles) de 2-hydroxy-4-iodobenzoate de méthyle, on obtient 2,42 g (71%) du produit attendu sous forme d'ester éthylique de point de fusion 74-5°C.

### (b) acide 2-hydroxy-4-(3,5,5,8,8-pentaméthyl-5,6,7,8-tétrahydro-2-naphtylthio) benzoïque.

De manière analogue à l'exemple 1(b) à partir de 2,4 g (6,1 mmoles) de l'ester éthylique précédent, on obtient 2,06 g (92%) d' acide 2-hydroxy-4-(3,5,5,8,8-pentaméthyl-5,6,7,8-tétrahydro-2-naphtylthio) benzoïque de point de fusion 197-200°C.

### EXEMPLE 19

*Acide 2-chloro-4-(3,5,5,8,8-pentaméthyl-5,6,7,8-tétrahydro-2-naphtylthio) benzoïque*.

### (a) acide 2-chloro-4-iodobenzoïque.

De manière analogue à l'exemple 14(a) à partir de 10 g (58,3 mmoles) d'acide 2-chloro-4-aminobenzoïque, on recueille 14,26 g (86%) d'acide 2-chloro-4-iodo benzoïque.

### (b) 2-chloro-4-iodobenzoate de méthyle.

De manière analogue à l'exemple 14(b) à partir de 13,9 g (49,2 mmoles) d'acide 2-chloro-4-iodobenzoïque, on obtient 11,52 g (79%) de l'ester méthylique attendu sous forme d'une huile.

### (c) 2-chloro-4-(3,5,5,8,8-pentaméthyl-5,6,7,8-tétrahydro-2-naphtylthio)benzoate d'éthyle.

De manière analogue à l'exemple 2(a) par réaction de 2 g (8,5 mmoles) de 3,5,5,8,8-pentaméthyl-5,6,7,8-tétrahydro-2-naphtylthiol avec 2,41 g (8,5 mmoles) de 2-chloro-4-iodobenzoate de méthyle, on obtient 1,25 g (35%) du produit attendu sous forme d'ester éthylique.

### (d) Acide 2-chloro-4-(3,5,5,8,8-pentaméthyl-5,6,7,8-tétrahydro-2-naphtylthio) benzoïque.

De manière analogue à l'exemple 1(b) à partir de 1,25 g (3 mmoles) de l'ester éthylique précédent, on obtient 1 g (96%) d' acide 2-chloro-4-(3,5,5,8,8-pentaméthyl-5,6,7,8-tétrahydro-2-naphtylthio) benzoïque de point de fusion 202-5°C.

### EXEMPLE 20

### Acide 4-(3-éthyl-5,6,7,8-tétrahydro-5,5,8,8-tétramethyl-2-naphtylthio) benzoïque.

### (a) O-3-éthyl-5,6,7,8-tétrahydro-5,5,8,8-tétramethyl-2-naphtyldimethylthiocarbamate

De manière analogue à l'exemple 8(a) par réaction de 5 g (21 mmoles) de 3-éthyl-5,6,7,8-tétrahydro-5,5,8,8-tétramethyl-2-naphtol avec 3,5 g (2,8 mmoles) de chlorure de diméthylthiocarbamoyle, on obtient 4,9 g (72%) du produit attendu de point de fusion 82-3°C.

### (b) S-3-éthyl-5,6,7,8-tétrahydro-5,5,8,8-tétramethyl-2-naphtyldimethylthiocarbamate.

De manière analogue à l'exemple 8(b) à partir de 4,5 g (14 mmoles) du produit précédent, on obtient 4,5 g (99%) de S-3-éthyl-5,6,7,8-tétrahydro-5,5,8,8-tétramethyl-2-naphtyldimethylthiocarbamate sous forme d'une huile marron.

### (c) 3-éthyl-5,6,7,8-tétrahydro-5,5,8,8-tétramethyl-2-naphtylthiol.

De manière analogue à l'exemple 8(c) à partir de 4,5 g (14 mmoles) du produit précédent, on obtient 2,9 g (84%) du thiol attendu sous forme d'une huile orangée.

### (d) 4-(3-éthyl-5,6,7,8-tétrahydro-5,5,8,8-tétramethyl-2-naphtylthio)benzoate d'éthyle.

De manière analogue à l'exemple 2(a) par réaction de 2 g (8,5 mmoles) de 3-éthyl-5,6,7,8-tétrahydro-5,5,8,8-tétramethyl-2-naphtylthiol avec 2,1 g (8 mmoles) de 4-iodobenzoate de méthyle, on obtient 1,9 g (64%) du produit attendu sous forme d'ester éthylique.

### (e) acide 4-(3-éthyl-5,6,7,8-tétrahydro-5,5,8,8-tétramethyl-2-naphtylthio)benzoïque.

De manière analogue à l'exemple 1(b) à partir de 1,9 g (5,1 mmoles) de l'ester éthylique précédent, on obtient 1,7 g (89%) d' acide 4-(3-éthyl-5,6,7,8-tétrahydro-5,5,8,8-tétramethyl-2-naphtylthio)benzoïque de point de fusion 232-5°C.

### EXEMPLE 21

### 4-(3-isopropyl-5,6,7,8-tétrahydro-5,5,8,8-tétramethyl-2-naphtylthio)benzoate d'éthyle.

### (a) O-3-isopropyl-5,6,7,8-tétrahydro-5,5,8,8-tétramethyl-2-naphtyldimethylthio carbamate

De manière analogue à l'exemple 8(a) par réaction de 5 g (20,3 mmoles) de 3-isopropyl-5,6,7,8-tétrahydro-5,5,8,8-tétramethyl-2-naphtol avec 3,26 g (26,4 mmoles) de chlorure de diméthylthiocarbamoyle, on obtient 2,26 g (33%) du produit attendu de point de fusion 99-100°C.

### (b) S-3-isopropyl-5,6,7,8-tétrahydro-5,5,8,8-tétramethyl-2-naphtyldimethylthio carbamate.

De manière analogue à l'exemple 8(b) à partir de 2,9 g (8,7 mmoles) du produit précèdent, on obtient 1,63 g (56%) de S-3-isopropyl-5,6,7,8-tétrahydro-5,5,8,8-tétramethyl-2-naphtyldimethylthiocarbamate de point de fusion 103-4°C.

### (c) 3-isopropyl-5,6,7,8-tétrahydro-5,5,8,8-tétramethyl-2-naphtylthiol.

De manière analogue à l'exemple 8(c) à partir de 4,5 g (14 mmoles) du produit précédent, on obtient 2,9 g (84%) du thiol attendu sous forme d'une huile orangée.

### (d) 4-(3-isopropyl-5,6,7,8-tétrahydro-5,5,8,8-tétramethyl-2-naphtylthio)benzoate d'éthyle.

De manière analogue à l'exemple 2(a) par réaction de 1,35 g (5,1 mmoles) de 3-isopropyl-5,6,7,8-tétrahydro-5,5,8,8-tétramethyl-2-naphtylthiol avec 1,1 g (5,1 mmoles) de 4-bromobenzoate de méthyle, on obtient 1,3 g (66%) du produit attendu sous forme d'ester éthylique.

### EXEMPLE 22

### 4-(3-n-propyl-5,6,7,8-tétrahydro-5,5,8,8-tétramethyl-2-naphtylthio)benzoate d'éthyle.

### (a) 3-n-propyl-5,6,7,8-tétrahydro-5,5,8,8-tétraméthyl-2-naphtol.

De manière analogue à l'exemple 7 (a) par réaction de 13,6 g (0,1 mole) de 2-n-propylphenol avec 18,3 g (0,1 mole) de 2,5-dichloro-2,5-diméthylhexane, on obtient après chromatographie sur colonne de silice élué avec du dichlorométhane 11,6 g (65%) du dérivé phénolique attendu de point de fusion 96-7°C.

### (b) O-3-n-propyl-5,6,7,8-tétrahydro-5,5,8,8-tétramethyl-2-naphtyldimethylthio carbamate.

De manière analogue à l'exemple 8(a) par réaction de 15 g (61 mmoles) de 3-propyl-5,6,7,8-tétrahydro-5,5,8,8-tétramethyl-2-naphtol avec 9,8 g (79 mmoles) de chlorure de diméthylthiocarbamoyle, on obtient 15,9 g (78%) de O-3-n-propyl-5,6,7,8-tétrahydro-5,5,8,8-tétramethyl-2-naphtyldimethylthio carbamate.

### (c) S-3-n-propyl-5,6,7,8-tétrahydro-5,5,8,8-tétramethyl-2-naphtyldimethylthio carbamate.

De manière analogue à l'exemple 8(b) à partir de 10 g (30 mmoles) du produit précédent, on obtient 6,8 g (68%) de S-3-n-propyl-5,6,7,8-tétrahydro-5,5,8,8-tétramethyl-2-naphtyldimethylthiocarbamate de point de fusion 102-4°C.

### (d) 3-n-propyl-5,6,7,8-tétrahydro-5,5,8,8-tétramethyl-2-naphtylthiol.

De manière analogue à l'exemple 8(c) à partir de 6,8 g (20,4 mmoles) du produit précédent, on obtient 5 g (93%) du thiol attendu sous forme d'une huile incolore.

### (e) 4-(3-n-propyl-5,6,7,8-tétrahydro-5,5,8,8-tétramethyl-2-naphtylthio)benzoate d'éthyle.

De manière analogue à l'exemple 2(a) par réaction de 2 g (7,6 mmoles) de 3-n-propyl-5,6,7,8-tétrahydro-5,5,8,8-tétramethyl-2-naphtylthiol avec 2 g (7,6 mmoles) de 4-iodobenzoate de méthyle, on obtient 2 g (65%) du produit attendu sous forme d'ester éthylique.

### EXEMPLE 23

### 4-(3,5,5,8,8-pentaméthyl-5,6,7,8-tétrahydro-2-naphtylthio)benzèneméthanol.

Dans un tricol et sous courant d'azote, on introduit 144 mg (3,8 mmoles) d'hydrure double de lithium et d'aluminium et 50 ml de THF. On introduit ensuite goutte à goutte une solution de 1,4 g (3,8 mmoles) de 4-(3,5,5,8,8-pentaméthyl-5,6,7,8-tétrahydro-2-naphtylthio)benzoate d'éthyle dans 25 ml de THF et chauffe à reflux pendant huit heures. L'excès d'hydrure est détruit avec une solution de tartrate double de sodium et de potassium, le milieu réactionnel est extrait avec de l'acétate d'éthyle. On décante la phase organique, sèche sur sulfate de magnésium, évapore. Le résidu obtenu est purifié par chromatographie sur colonne de silice élué avec un mélange d'heptane et d'acétate d'éthyle (90-10). On recueille 660 mg (51%) de l'alcool attendu de point de fusion 81-2°C.

### EXEMPLE 24

### 4-(3,5,5,8,8-pentaméthyl-5,6,7,8-tétrahydro-2-naphtylthio)benzaldéhyde.

Dans un tricol et sous courant d'azote, on introduit 60 µl (0,7 mmole) de chlorure d'oxalyle et 5 ml de dichlorométhane. A -60°C, on ajoute 100 µl (1,4 mmole) de DMSO et agite 10' puis ajoute une solution de 200 mg (0,6 mmole) de 4-(3,5,5,8,8-pentaméthyl-5,6,7,8-tétrahydro-2-naphtylthio)benzèneméthanol dans 5 ml de dichlorométhane et 400 µl (3 mmoles) de triéthylamine. On agite à température ambiante pendant une heure, verse le milieu réactionnel dans l'eau, extrait avec du dichlorométhane, décante la phase organique, sèche sur sulfate de magnésium, évapore. Le résidu obtenu est purifié par chromatographie sur colonne de silice élué avec un mélange d'heptane et d'acétate d'éthyle (80-20). On recueille 145 mg (73%) de l'aldehyde attendu de point de fusion 98-9°C.

### EXEMPLE 25

### N-éthyl 4-(3,5,5,8,8-pentaméthyl-5,6,7,8-tétrahydro-2-naphtylthio)benzamide.

### (a) chlorure de 4-(3,5,5,8,8-pentaméthyl-5,6,7,8-tétrahydro-2-naphtylthio)benzoyle.

Dans un ballon, on introduit une solution de 1,4 g (4 mmoles) d'acide 4-(3,5,5,8,8-pentaméthyl-5,6,7,8-tétrahydro-2-naphtylthio)benzoïque dans 100 ml de dichlorométhane anhydre ajoute 900 µl (4,4 mmoles) de dicyclohexylamine et agite pendant une heure. On ajoute ensuite 320 µl (4,4 mmoles) de chlorure de thionyle et agite une heure. On évapore à sec, reprend par l'éther éthylique anhydre, filtre le sel de dicyclohexylamine et évapore le filtrat. On recueille 1,5 g (100%) du chlorure d'acide brut qui sera utilisé tel quel pour la suite de la synthèse.

### (b) N-éthyl 4-(3,5,5,8,8-pentaméthyl-5,6,7,8-tétrahydro-2-naphtylthio)benzamide.

Dans un ballon, on introduit 640 µL (8 mmoles) d'une solution d'éthylamine à 70% et 20 ml de THF. On ajoute goutte à goutte une solution de 735 mg (2 mmoles) de chlorure de 4-(3,5,5,8,8-pentaméthyl-5,6,7,8-tétrahydro-2-naphtylthio)benzoyle dissous dans 30 ml de THF et agite à température ambiante trois heures. On verse le milieu réactionnel dans l'eau, extrait avec de l'acétate d'éthyle, décante la phase organique, sèche sur sulfate de magnésium, évapore. Le résidu obtenu est purifié par chromatographie sur colonne de silice élué avec un mélange d'heptane et d'acétate d'éthyle (90-10). On recueille après évaporation des solvants 307 mg (41%) de l'amide éthylique attendu de point de fusion 223-4°C.

### EXEMPLE 26

### N-4-hydroxyphényl-4-(3,5,5,8,8-pentaméthyl-5,6,7,8-tétrahydro-2-naphtyl thio)benzamide.

### (a) N-4-acétoxyphényl-4-(3,5,5,8,8-pentaméthyl-5,6,7,8-tétrahydro-2-naphtyl thio)benzamide.

De manière analogue à l'exemple 25(b) par réaction de 735 mg (2 mmoles) de chlorure de 4-(3,5,5,8,8-pentaméthyl-5,6,7,8-tétrahydro-2-naphtylthio)benzoyle avec 900 mg (6 mmoles) d'acétate de 4-aminobenzyle, on obtient 250 mg (26%) de l'amide attendu de point de fusion 186-7°C.

### (b) N-4-hydroxyphényl-4-(3,5,5,8,8-pentaméthyl-5,6,7,8-tétrahydro-2-naphtyl thio)benzamide.

De manière analogue à l'exemple 2(a) à partir de 100 mg (0,2 mmole) de N-4-acétoxyphényl-4-(3,5,5,8,8-pentaméthyl-5,6,7,8-tétrahydro-2-naphtylthio) benzamide, on obtient 65 mg (71%) du produit attendu de point de fusion 236-7°C.

### EXEMPLE 27

Dans cet exemple, on a illustré diverses formulations concrètes à base des composés selon l'invention.

### A- VOIE ORALE

| (a) Comprimé de 0,2 g | |
|---|---|
| - Composé préparé à l'exemple 7 | 0,001 g |
| - Amidon | 0,114 g |
| - Phosphate bicalcique | 0,020 g |
| - Silice | 0,020 g |
| - Lactose | 0,030 g |
| - Talc | 0,010 g |
| - Stéarate de magnésium | 0,005 g |

| (b) Suspension buvable en ampoules de 10 ml | |
|---|---|
| - Composé de l'exemple 8 | 0,05 g |
| - Glycérine | 1,000g |
| - Sorbitol à 70% | 1,000g |
| - Saccharinate de sodium | 0,010 g |
| - Parahydroxybenzoate de méthyle | 0,080 g |
| - Arome qs | |
| - Eau purifiée qsp | 10ml |

### B- VOIE TOPIQUE

| (a) Onguent | |
|---|---|
| - Composé de l'exemple 8 | 0,020 g |
| - Myristate d'isopropyle | 81,700 g |
| - Huile de vaseline fluide | 9,100 g |
| - Silice ("Aérosil 200" vendue par DEGUSSA) | 9,180 g |

| (b) Onguent | |
|---|---|
| - Composé de l'exemple 9 | 0,300 g |
| - Vaseline blanche code qsp | 100 g |

| (c) Crème Eau-dans-Huile non ionique | |
|---|---|
| - Composé de l'exemple 7 | 0,100 g |
| - Mélange d'alcools de lanoline émulsifs, de cires et d'huiles ("Eucerine anhydre" vendu par BDF) | 39,900 g |
| - Parahydroxybenzoate de méthyle | 0,075 g |
| - Parahydroxybenzoate de propyle | 0,075 g |
| - Eau déminéralisée stérile qsp | 100 g |

| (d) Lotion | |
|---|---|
| - Composé de l'exemple 8 | 0,100 g |
| - Polyéthylène glycol (PEG 400) | 69,900 g |
| - Ethanol à 95% | 30,000 g |

| (e) Onguent hydrophobe | |
|---|---|
| - Composé de l'exemple 7 | 0,300 g |
| - Mirystate d'isopropyle | 36,400 g |
| - Huile de silicone ("Rhodorsil 47 V 300" vendu par RHONE-POULENC) | 36,400 g |
| - Cire d'abeille | 13,600 g |
| - Huile de silicone ("Abil 300.000 cst" vendu par GOLDSCHMIDT) qsp | 100g |

| (f) Crème Huile-dans-Eau non ionique | |
|---|---|
| - Composé de l'exemple 7 | 0,500 g |
| - Alcool cétylique | 4,000 g |
| - Monostéarate de glycérole | 2,500 g |
| - Stéarate de PEG 50 | 2,500 g |
| - Beurre de karité | 9,200 g |
| - Propylène glycol | 2,000 g |
| - Parahydroxybenzoate de méthyle | 0,075 g |
| - Parahydroxybenzoate de propyle | 0,075 g |
| - Eau déminéralisée stérile qsp | 100 g |

## Revendications

1. Composés bicycliques-aromatiques, caractérisés par le fait qu'ils répondent à la formule générale (I) suivante : 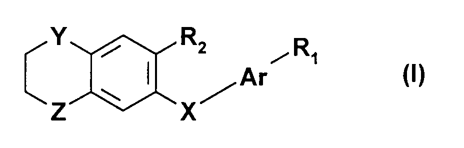 dans laquelle :
∗ R₁ représente un atome d'hydrogène, un radical -CH₃, un radical -CH₂-O-R₃, un radical -CH₂-O-CO-R₄, un radical -O-R₅, un radical -O-(CH₂)ₘ-(CO)ₙ-R₆, un radical -CO-R₇, un radical -CO-O-R₈, ou encore un radical -S(O)ₚ-R₉, les valeurs m, n et p ainsi que les différents radicaux R₃ à R₉ ayant la signification donnée ci-après,
∗ R₂ représente un atome d'hydrogène ou un atome d'halogène, un radical alkyle inférieur de C₁-C₆, un radical -NO₂, un radical -O-COR₄, un radical -OR₉ ou bien encore un radical 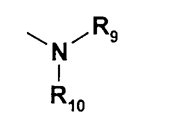 les radicaux R₄, R₉, et R₁₀ ayant la signification donnée ci-après,
* Ar représente un radical choisi parmi les radicaux de formules (a)-(e) suivantes : 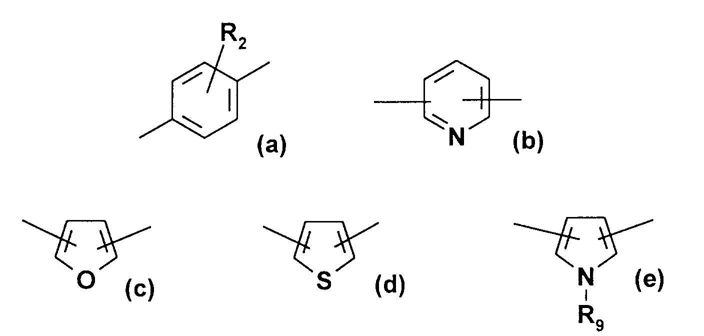 dans lesquelles R₂ a la signification ci-dessus, et R₉ celle donnée ci-après,
∗ X représente -O-, -S(O)ₜ- ou un radical -NR₉-, la valeur t et le radical R₉ ayant la signification donnée ci-après,
* Y et Z représentent -O-, -S(O)ₜ- ou encore un radical -CR₁₁R₁₂, la valeur t et les radicaux R₁₁ et R₁₂ ayant la signification donnée ci-après,
étant entendu que dans tout ce qui précède :
- m est un nombre entier égal à 1, 2 ou 3, n est un nombre entier égal à 0 ou 1, p est un nombre entier égal à 0, 1, 2 ou 3 et t est un nombre entier égal à 0, 1 ou 2,
- R₃ représente un atome d'hydrogène ou un radical alkyle inférieur de C₁-C₆,
- R₄ représente un radical alkyle inférieur de C₁-C₆,
- R₅ représente un atome d'hydrogène ou un radical alkyle inférieur de C₁-C₆,
- R₆ représente un radical alkyle inférieur de C₁-C₆ ou un hétérocycle,
- R₇ représente un atome d'hydrogène, un radical alkyle inférieur de C₁-C₆ ou un radical : 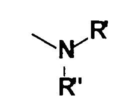 dans lequel R' et R'', identiques ou différents, représentent un atome d'hydrogène, un radical alkyle inférieur de C₁-C₆, un radical mono ou polyhydroxyalkyle, un radical aryle éventuellement substitué ou un reste d'aminoacide ou de peptide ou de sucre ou encore, pris ensemble, forment un hétérocycle,
- R₈ représente un atome d'hydrogène, un radical alkyle linéaire ou ramifié ayant de 1 à 20 atomes de carbone, un radical alkényle, un radical mono ou polyhydroxyalkyle, un radical aryle ou aralkyle éventuellement substitué ou un reste de sucre ou un reste d'aminoacide ou de peptide,
- R₉ représente un atome d'hydrogène ou un radical alkyle inférieur de C₁-C₆,
- R₁₀ représente un atome d'hydrogène ou un radical alkyle inférieur de C₁-C₆,
- R₁₁ représente un atome d'hydrogène ou un radical alkyle inférieur de C₁-C₆,
- R₁₂ représente un atome d'hydrogène ou un radical alkyle inférieur de C₁-C₆,
- Y et Z ne peuvent représenter en même temps un atome d'oxygène ou un radical -S(O)ₜ-.
ainsi que les sels et les analogues chiraux desdits composés de formule (I).

2. Composés selon la revendication 1, caractérisés en ce qu'ils se présentent sous forme de sels d'un métal alcalin ou alcalino-terreux, ou encore de zinc ou d'une amine organique.

3. Composés selon l'une des revendications 1 ou 2, caractérisés en ce que les radicaux alkyles inférieurs sont choisis dans le groupe constitué par les radicaux méthyle, éthyle, isopropyle, butyle, tertiobutyle et hexyle.

4. Composés selon l'une quelconque des revendications précédentes, caractérisés en ce que les radicaux alkyles linéaires ou ramifiés ayant de 1 à 20 atomes de carbone sont choisis dans le groupe constitué par les radicaux méthyle, éthyle, propyle, 2-éthyl-hexyle, octyle, dodécyle, hexadécyle et octadécyle.

5. Composés selon l'une quelconque des revendications précédentes, caractérisés en ce que les radicaux monohydroxyalkyles sont choisis dans le groupe constitué par les radicaux 2-hydroxyéthyle, 2-hydroxypropyle ou 3-hydroxypropyle.

6. Composés selon l'une quelconque des revendications précédentes, caractérisés en ce que les radicaux polyhydroxyalkyles sont choisis dans le groupe constitué par les radicaux 2,3-dihydroxypropyle, 2,3,4-trihydroxybutyle, 2,3,4,5-tétrahydroxypentyle ou le reste du pentaérythritol.

7. Composés selon l'une quelconque des revendications précédentes, caractérisés en ce que le radical aryle est un radical phényle éventuellement substitué par au moins un atome d'halogène, un hydroxyle ou une fonction nitro.

8. Composés selon l'une quelconque des revendications précédentes, caractérisés en ce que les radicaux aralkyles sont choisis dans le groupe constitué par les radicaux benzyle ou phénéthyle éventuellement substitués par au moins un atome d'halogène, un hydroxyle ou une fonction nitro.

9. Composés selon l'une quelconque des revendications précédentes, caractérisés en ce que les radicaux alkényles sont choisis dans le groupe constitué par les radicaux contenant de 2 à 5 atomes de carbone et présentant une ou plusieurs insaturations éthyléniques, en particulier le radical allyle.

10. Composés selon l'une quelconque des revendications précédentes, caractérisés en ce que les restes de sucre sont choisis dans le groupe constitué par les restes de glucose, de galactose, de mannose ou d'acide glucuronique.

11. Composés selon l'une quelconque des revendications précédentes, caractérisés en ce que les restes d'aminoacide sont choisis dans le groupe constitué par les restes dérivant de la lysine, de la glycine ou de l'acide aspartique.

12. Composés selon l'une quelconque des revendications précédentes, caractérisés en ce que les restes de peptides sont choisis dans le groupe constitué par les restes de dipeptides ou de tripeptides.

13. Composés selon l'une quelconque des revendications précédentes, caractérisés en ce que les radicaux hétérocycliques sont choisis dans le groupe constitué par les radicaux pipéridino, morpholino, pyrrolidino ou pipérazino, éventuellement substitués en position 4 par un radical alkyle en C₁-C₆ ou mono ou polyhydroxyalkyle.

14. Composés selon l'une quelconque des revendications précédentes, caractérisés en ce que les atomes d'halogène sont choisis dans le groupe constitué par le fluor, le chlore et le brome.

15. Composés selon la revendication 1, caractérisés par le fait qu'ils sont pris, seuls ou en mélanges, dans le groupe constitué par :
Acide 4-(5,6,7,8-tétrahydro-5,5,8,8-tétraméthyl-2-naphthyloxy)benzoïque
Acide 4-(5,6,7,8-tétrahydro-5,5,8,8-tétraméthyl-2-naphthylthio)benzoïque
Acide 4-(5,6,7,8-tétrahydro-5,5,8,8-tétraméthyl-2 naphthylsulfinyl) benzoïque
Acide 4-(5,6,7,8-tétrahydro-5,5,8,8-tétraméthyl-2-naphthylsulfonyl) benzoïque
Acide 4-(5,6,7,8-tétrahydro-5,5,8,8-tétraméthyl-2-naphthylamino)benzoïque
Acide 5-(5,6,7,8-tétrahydro-5,5,8,8-tétraméthyl-2-naphthylthio)-2-thiophene carboxylique
Acide 4-(3,5,5,8,8-pentaméthyl-5,6,7,8-tétrahydro-2-naphthyloxy) benzoïque
Acide 4-(3,5,5,8,8-pentaméthyl-5,6,7,8-tétrahydro-2-naphthylthio) benzoïque
Acide 4-(3-éthyl-5,5,8,8-tétraméthyl-5,6,7,8-tétrahydro-2-naphthyloxy) benzoïque
Acide 4-(3-isopropyl-5,5,8,8-tétraméthyl-5,6,7,8-tétrahydro-2-naphthyloxy) benzoïque
4-(5,6,7,8-tétrahydro-5,5,8,8-tétraméthyl-2-naphthyloxy)acétophenone
4-(5,6,7,8-tétrahydro-5,5,8,8-tétraméthyl-2-naphthyloxy)benzaldéhyde
Acide 4-(3-bromo-5,6,7,8-tétrahydro-5,5,8,8-tétramethyl-2-naphtyloxy) benzoïque
Acide 3-méthyl-4-(5,6,7,8-tétrahydro-5,5,8,8-tétramethyl-2-naphtylthio) benzoïque
Acide 3-méthyl-4-(3,5,5,8,8-pentaméthyl-5,6,7,8-tétrahydro-2-naphtylthio)benzoïque
Acide 3-méthyl-4-(3-éthyl-5,6,7,8-tétrahydro-5,5,8,8-tétramethyl-2-naphtyloxy) benzoïque
Acide 6-(3,5,5,8,8-pentaméthyl-5,6,7,8-tétrahydro-2-naphtylthio)nicotinique
Acide 2-hydroxy-4-(3,5,5,8,8-pentaméthyl-5,6,7,8-tétrahydrol-2-naphtylthio) benzoïque
Acide 2-chloro-4-(3,5,5,8,8-pentaméthyl-5,6,7,8-tétrahydro-2-naphtylthio) benzoïque
Acide 4-(3-éthyl-5,6,7,8-tétrahydro-5,5,8,8-tétramethyl-2-naphtylthio) benzoïque
Acide 4-(3-isopropyl-5,6,7,8-tétrahydro-5,5,8,8-tétramethyl-2-naphtylthio) benzoïque
Acide 4-(3-n-propyl-5,6,7,8-tétrahydro-5,5,8,8-tétramethyl-2-naphtylthio) benzoïque
4-(3,5,5,8,8-pentaméthyl-5,6,7,8-tétrahydro-2-naphtylthio)benzèneméthanol
4-(3,5,5,8,8-pentaméthyl-5,6,7,8-tétrahydro-2-naphtylthio)benzaldéhyde
N-éthyl-4-(3,5,5,8,8-pentaméthyl-5,6,7,8-tétrahydro-2-naphtylthio)benzamide
N-4-hydroxyphényl-4-(3,5,5,8,8-pentaméthyl-5,6,7,8-tétrahydro-2-naphtyl thio)benzamide.

16. Composés selon la revendication 1, caractérisés par le fait qu'ils présentent l'une au moins, et de préférence toutes, les caractéristiques suivantes : R₁ est un radical -CO-R₇ ; R₂ est un radical alkyl inférieur ou un radical -OR₉ ; Ar représente un radical de formule (a) ; et X représente -O-, -S- ou -NR₉-.

17. Composés selon l'une quelconque des revendications précédentes pour une utilisation comme médicament.

18. Composés selon la revendication 17 pour une utilisation comme médicament destiné au traitement des affections dermatologiques, rhumatismales, respiratoires, cardiovasculaires et ophtalmologiques.

19. Utilisation de l'un au moins des composés définis aux revendications 1 à 16 pour la fabrication d'un médicament destiné au traitement des affections dermatologiques, rhumatismales, respiratoires, cardiovasculaires et ophtalmologiques.

20. Composition pharmaceutique, caractérisée par le fait qu'elle comprend, dans un support pharmaceutiquement acceptable, au moins l'un des composés tels que définis à l'une quelconque des revendications 1 à 16.

21. Composition selon la revendication 20, caractérisée en ce que la concentration en composé(s) selon l'une des revendication 1 à 16 est comprise entre 0,001 % et 5 % en poids par rapport à l'ensemble de la composition.

22. Composition cosmétique, caractérisée par le fait qu'elle comprend, dans un support cosmétiquement acceptable, au moins l'un des composés tels que définis à l'une quelconque des revendications 1 à 16.

23. Composition selon la revendication 22, caractérisée en ce que la concentration en composé(s) selon l'une des revendications 1 à 16 est comprise entre 0,001 % et 3 % en poids par rapport à l'ensemble de la composition.

24. Utilisation d'une composition cosmétique telle que définie à l'une des revendications 22 ou 23 pour l'hygiène corporelle ou capillaire.

## Claims

1. Bicyclic-aromatic compounds, characterized by the fact that they correspond to the following general formula (I): 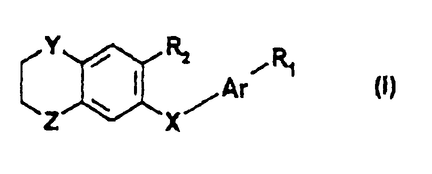 in which:
* R₁ represents a hydrogen atom, a -CH₃ radical, a -CH₂-O-R₃ radical, a -CH₂-O-CO-R₄ radical, an -O-R₅ radical, an -O-(CH₂)ₘ-(CO)ₙ-R₆ radical, a -CO-R₇ radical, a -CO-O-R₈ radical or alternatively an -S(O)ₚ-R₉ radical, the values m, n and p as well as the various radicals R₃ to R₉ having the meaning given below,
* R₂ represents a hydrogen atom or a halogen atom, a lower C₁-C₆ alkyl radical, an -NO₂ radical, a -O-COR₄ radical, an -OR₉ radical or alternatively a radical 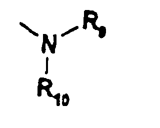 the radicals R₄, R₉ and R₁₀ having the meaning given below,
* Ar represents a radical chosen from the following radicals of formulae (a)-(e) 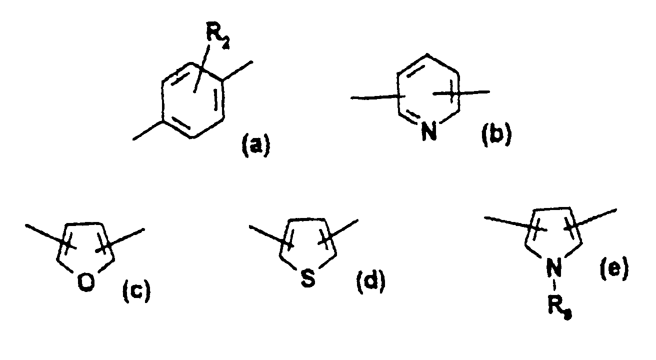 in which R₂ has the above meaning, and R₉ that given below,
* X represents -O-, -S(O)ₜ- or an -NR₉- radical, the value t and the radical R₉ having the meaning given below,
* Y and Z represent -O-, -S(O)ₜ- or alternatively a radical -CR₁₁R₁₂, the value t and the radicals R₁₁ and R₁₂ having the meaning given below,
it being understood that in all that precedes:
- m is an integer equal to 1, 2 or 3, n is an integer equal to 0 or 1, p is an integer equal to 0, 1, 2 or 3 and t is an integer equal to 0, 1 or 2,
- R₃ represents a hydrogen atom or a lower C₁-C₆ alkyl radical,
- R₄ represents a lower C₁-C₆ alkyl radical,
- R₅ represents a hydrogen atom or a lower C₁-C₆ alkyl radical,
- R₆ represents a lower C₁-C₆ alkyl radical or a heterocycle,
- R₇ represents a hydrogen atom, a lower C₁-C₆ alkyl radical or a radical: 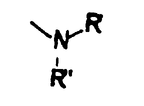 in which R' and R'', which may be identical or different, represent a hydrogen atom, a lower C₁-C₆ alkyl radical, a mono- or polyhydroxy-alkyl radical, an optionally substituted aryl radical or an amino acid or peptide or sugar residue, or alternatively, taken together, form a heterocycle,
- R₈ represents a hydrogen atom, a linear or branched alkyl radical having 1 to 20 carbon atoms, an alkenyl radical, a mono- or polyhydroxyalkyl radical, an optionally substituted aryl or aralkyl radical or a sugar residue or an amino acid or peptide residue,
- R₉ represents a hydrogen atom or a lower C₁-C₆ alkyl radical,
- R₁₀ represents a hydrogen atom or a lower C₁-C₆ alkyl radical,
- R₁₁ represents a hydrogen atom or a lower C₁-C₆ alkyl radical,
- R₁₂ represents a hydrogen atom or a lower C₁-C₆ alkyl radical,
- Y and Z cannot represent at the same time an oxygen atom or an -S(O)ₜ- radical.
as well as the salts and the chiral analogs of the said compounds of formula (I).

2. Compounds according to Claim 1, characterized in that they are in the form of salts of an alkali or alkaline-earth metal, or alternatively of zinc or of an organic amine.

3. Compounds according to one of Claims 1 and 2, characterized in that the lower alkyl radicals are chosen from the group consisting of methyl, ethyl, isopropyl, butyl, tert-butyl and hexyl radicals.

4. Compounds according to any one of the preceding claims, characterized in that the linear or branched alkyl radicals having 1 to 20 carbon atoms are chosen from the group consisting of methyl, ethyl, propyl, 2-ethylhexyl, octyl, dodecyl, hexadecyl and octadecyl radicals.

5. Compounds according to any one of the preceding claims, characterized in that the monohydroxyalkyl radicals are chosen from the group consisting of 2-hydroxyethyl, 2-hydroxypropyl or 3-hydroxypropyl radicals.

6. Compounds according to any one of the preceding claims, characterized in that the polyhydroxyalkyl radicals are chosen from the group consisting of 2,3-dihydroxypropyl, 2,3,4-trihydroxybutyl or 2,3,4,5-tetrahydroxypentyl radicals or the pentaerythritol residue.

7. Compounds according to any one of the preceding claims, characterized in that the aryl radical is a phenyl radical optionally substituted by at least one halogen atom, a hydroxyl or a nitro functional group.

8. Compounds according to any one of the preceding claims, characterized in that the aralkyl radicals are chosen from the group consisting of benzyl or phenethyl radicals optionally substituted by at least one halogen atom, a hydroxyl or a nitro functional group.

9. Compounds according to any one of the preceding claims, characterized in that the alkenyl radicals are chosen from the group consisting of the radicals containing 2 to 5 carbon atoms and having one or more ethylenic unsaturations, in particular the allyl radical.

10. Compounds according to any one of the preceding claims, characterized in that the sugar residues are chosen from the group consisting of glucose, galactose, mannose or glucuronic acid residues.

11. Compounds according to any one of the preceding claims, characterized in that the amino acid residues are chosen from the group consisting of residues derived from lysine, glycine or aspartic acid.

12. Compounds according to any one of the preceding claims, characterized in that the peptide residues are chosen from the group consisting of dipeptide or tripeptide residues.

13. Compounds according to any one of the preceding claims, characterized in that the heterocyclic radicals are chosen from the group consisting of piperidino, morpholino, pyrrolidino or piperazino radicals which are optionally substituted at position 4 by a C₁-C₆ alkyl radical or a mono- or polyhydroxyalkyl radical.

14. Compounds according to any one of the preceding claims, characterized in that the halogen atoms are chosen from the group consisting of fluorine, chlorine and bromine.

15. Compounds according to Claim 1, characterized by the fact that they are chosen, alone or in the form of mixtures, from the group consisting of:
4-(5,6,7,8-tetrahydro-5,5,8,8-tetramethyl-2-naphthyloxy)benzoic acid
4-(5,6,7,8-tetrahydro-5,5,8,8-tetramethyl-2-naphthylthio)benzoic acid
4-(5,6,7,8-tetrahydro-5,5,8,8-tetramethyl-2-naphthylsulphinyl)benzoic acid
4-(5,6,7,8-tetrahydro-5,5,8,8-tetramethyl-2-naphthylsulphonyl)benzoic acid
4-(5,6,7,8-tetrahydro-5,5,8,8-tetramethyl-2-naphthylamino)benzoic acid
5-(5,6,7,8-tetrahydro-5,5,8,8-tetramethyl-2-naphthylthio)-2-thiophene carboxylic acid
4-(3,5,5,8,8-pentamethyl-5,6,7,8-tetrahydro-2-naphthyloxy)benzoic acid
4-(3,5,5,8,8-pentamethyl-5,6,7,8-tetrahydro-2-naphthylthio)benzoic acid
4-(3-ethyl-5,5,8,8-tetramethyl-5,6,7,8-tetrahydro-2-naphthyloxy)benzoic acid
4-(3-isopropyl-5,5,8,8-tetramethyl-5,6,7,8-tetrahydro-2-naphthyloxy)benzoic acid
4-(5,6,7,8-tetrahydro-5,5,8,8-tetramethyl-2-naphthyloxy)acetophenone
4-(5,6,7,8-tetrahydro-5,5,8,8-tetramethyl-2-naphthyloxy)benzaldehyde
4-(3-bromo-5,6,7,8-tetrahydro-5,5,8,8-tetramethyl-2-naphthyloxy)benzoic acid
3-methyl-4-(5,6,7,8-tetrahydro-5,5,8,8-tetramethyl-2-naphthylthio)benzoic acid
3-methyl-4-(3,5,5,8,8-pentamethyl-5,6,7,8-tetrahydro-2-naphthylthio)benzoic acid
3-methyl-4-(3-ethyl-5,6,7,8-tetrahydro-5,5,8,8-tetramethyl-2-naphthyloxy)benzoic acid
6-(3,5,5,8,8-pentamethyl-5,6,7,8-tetrahydro-2-naphthylthio)nicotinic acid
2-hydroxy-4-(3,5,5,8,8-pentamethyl-5,6,7,8-tetrahydrol-2-naphthylthio)benzoic acid
2-chloro-4-(3,5,5,8,8-pentamethyl-5,6,7,8-tetrahydro-2-naphthylthio)benzoic acid
4-(3-ethyl-5,6,7,8-tetrahydro-5,5,8,8-tetramethyl-2-naphthylthio)benzoic acid
4-(3-isopropyl-5,6,7,8-tetrahydro-5,5,8,8-tetramethyl-2-naphthylthio)benzoic acid
4-(3-n-propyl-5,6,7,8-tetrahydro-5,5,8,8-tetramethyl-2-naphthylthio)benzoic acid
4-(3,5,5,8,8-pentamethyl-5,6,7,8-tetrahydro-2-naphthylthio)benzenemethanol
4-(3,5,5,8,8-pentamethyl-5,6,7,8-tetrahydro-2-naphthylthio)benzaldehyde
N-ethyl-4-(3,5,5,8,8-pentamethyl-5,6,7,8-tetrahydro-2-naphthylthio)benzamide
N-4-hydroxyphenyl-4-(3,5,5,8,8-pentamethyl-5,6,7,8-tetrahydro-2-naphthylthio)benzamide

16. Compounds according to Claim 1, characterized by the fact that they exhibit at least one, and preferably all, of the following characteristics: R₁ is a -CO-R₇ radical; R₂ is a lower alkyl radical or an -OR₉ radical; Ar represents a radical of formula (a); and X represents -O-, -S- or -NR₉-.

17. Compounds according to any one of the preceding claims, for use as medicinal products.

18. Compounds according to Claim 17, for use as medicinal products intended for the treatment of dermatological, rheumatic, respiratory, cardiovascular and ophthalmological conditions.

19. Use of at least one of the compounds defined in Claims 1 to 16 for the manufacture of a medicinal product intended for the treatment of dermatological, rheumatic, respiratory, cardiovascular and ophthalmological conditions.

20. Pharmaceutical composition, characterized by the fact that it comprises, in a pharmaceutically acceptable carrier, at least one of the compounds as defined in any one of Claims 1 to 16.

21. Composition according to Claim 20, characterized in that the concentration of compound(s) according to one of Claims 1 to 16 is between 0.001 % and 5 % by weight relative to the whole composition.

22. Cosmetic composition, characterized by the fact that it comprises, in a cosmetically acceptable carrier, at least one of the compounds as defined in any one of Claims 1 to 16.

23. Composition according to Claim 22, characterized in that the concentration of compound(s) according to one of Claims 1 to 16 is between 0.001 % and 3 % by weight relative to the whole composition.

24. Use of a cosmetic composition as defined in one of Claims 22 and 23 for body or hair care.

## Patentansprüche

1. Bicyclische aromatische Verbindungen,
**dadurch gekennzeichnet, daß**
sie der folgenden allgemeinen Formel (I) entsprechen: 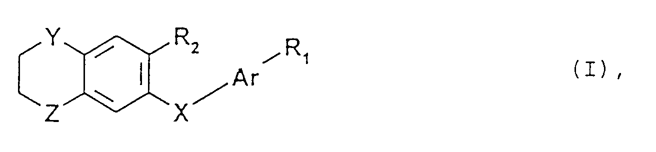 worin bedeuten:
* R₁ Wasserstoff, die Gruppe -CH₃, eine Gruppe -CH₂-O-R₃, eine Gruppe -CH₂-Q-CO-R₄, eine Gruppe - O-R₅, eine Gruppe -O-(CH₂)ₘ-(CO)ₙ-R₆, eine Gruppe -CO-R₇, eine Gruppe -CO-O-R₈ oder auch eine Gruppe -S(O)ₚ-R₉, wobei die Werte m, n und p sowie die verschiedenen Gruppen R₃ bis R₉ die nachstehend angegebenen Bedeutungen aufweisen,
* R² ein Wasserstoff- oder Halogenatom, eine niedere Alkylgruppe mit 1 bis 6 Kohlenstoffatomen, die Gruppe -NO₂, eine Gruppe -O-COR₄, eine Gruppe -OR₉ oder auch eine Gruppe 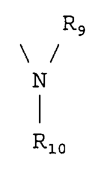 wobei R₄, R₉ und R₁₀ die nachfolgend angegebene Bedeutung aufweisen,
* Ar eine Gruppe, die unter den Gruppen der folgenden Formeln (a) bis (e) ausgewählt ist: 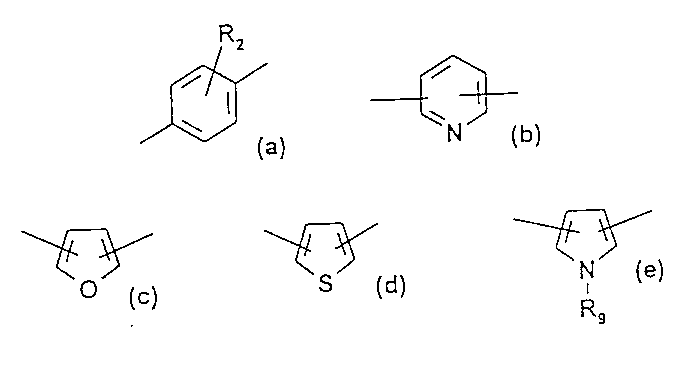 worin R₂ die oben angegebene Bedeutung und R₉ die nachfolgend angegebene Bedeutung aufweisen,
* X -O-, -S(O)ₜ- oder -NR₉-, wobei der Wert t und die Gruppe R₉ die nachfolgend angegebene Bedeutung aufweisen,
* Y und Z -O-, -S(O)ₜ oder auch -CR₁₁R₁₂, wobei der Wert t und die Gruppen R₁₁ und R₁₂ die nachfolgend angegebene Bedeutung aufweisen,
mit der Maßgabe, daß im folgenden bedeuten:
- m 1, 2 oder 3, n Null oder 1, p Null, 1, 2 oder 3 und t Null, 1 oder 2,
- R₃ Wasserstoff oder eine niedere Alkylgruppe mit 1 bis 6 Kohlenstoffatomen
- R₄ eine niedere Alkylgruppe mit 1 bis 6 Kohlenstoffatomen,
- R₅ Wasserstoff oder eine niedere Alkylgruppe mit 1 bis 6 Kohlenstoffatomen,
- R₆ eine niedere Alkylgruppe mit 1 bis 6 Kohlenstoffatomen oder einen Heterocyclus,
- R₇ Wasserstoff, eine niedere Alkylgruppe mit 1 bis 6 Kohlenstoffatomen oder eine Gruppe 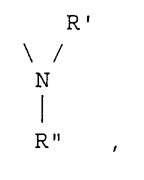 worin R' und R'', die identisch oder voneinander verschieden sind, Wasserstoff, eine niedere Alkylgruppe mit 1 bis 6 Kohlenstoffatomen, eine Mono- oder Polyhydroxyalkylgruppe, eine Arylgruppe, die gegebenenfalls substituiert ist, oder einen Aminosäure-, Petid- oder Zuckerrest bedeuten oder gemeinsam einen Heterocyclus bilden,
- R₈ Wasserstoff, eine geradkettige oder verzweigte Alkylgruppe mit 1 bis 20 Kohlenstoffatomen, eine Alkenylgruppe, eine Mono- oder Polyhydroxyalkylgruppe, eine Aryl- oder Aralkylgruppe, die gegebenenfalls substituiert sind, oder einen Zucker-, Aminosäure- oder Peptidrest,
- R₉ Wasserstoff oder eine niedere Alkylgruppe mit 1 bis 6 Kohlenstoffatomen,
- R₁₀ Wasserstoff oder eine niedere Alkylgruppe mit 1 bis 6 Kohlenstoffatomen,
- R₁₁ Wasserstoff oder eine niedere Alkylgruppe mit 1 bis 6 Kohlenstoffatomen,
- R₁₂ Wasserstoff oder eine niedere Alkylgruppe mit 1 bis 6 Kohlenstoffatomen,
- wobei Y und Z nicht gleichzeitig Sauerstoff oder eine Gruppe -S(O)ₜ bedeuten können,
sowie die Salze und chiralen Analoga der Verbindungen der Formel (I).

2. Verbindungen nach Anspruch 1, dadurch gekennzeichnet, daß sie in Form von Alkali- oder Erdalkalimetallsalzen oder auch Zinksalzen oder Salzen anorganischer Amine vorliegen.

3. Verbindungen nach einem der Ansprüche 1 oder 2, dadurch gekennzeichnet, daß die niederen Alkylgruppen unter den Gruppen Methyl, Ethyl, Isopropyl, Butyl, *tert*.-Butyl und Hexyl ausgewählt sind.

4. Verbindungen nach einem der vorhergehenden Ansprüche, dadurch gekennzeichnet, daß die geradkettigen oder verzweigten Alkylgruppen mit 1 bis 20 Kohlenstoffatomen unter den Gruppen Methyl, Ethyl, Propyl, 2-Ethylhexyl, Octyl, Dodecyl, Hexadecyl und Octadecyl ausgewählt sind.

5. Verbindungen nach einem der vorhergehenden Ansprüche, dadurch gekennzeichnet, daß die Monohydroxyalkylgruppen unter den Gruppen 2-Hydroxyethyl, 2-Hydroxypropyl oder 3-Hydroxypropyl ausgewählt sind.

6. Verbindungen nach einem der vorhergehenden Ansprüche, dadurch gekennzeichnet, daß die Polyhydroxyalkylgruppen unter den Gruppen 2,3-Dihydroxypropyl, 2,3,4-Trihydroxybutyl, 2,3,4,5-Tetrahydroxypentyl oder Pentaerythrit ausgewählt sind.

7. Verbindungen nach einem der vorhergehenden Ansprüche, dadurch gekennzeichnet, daß die Arylgruppe eine Phenylgruppe ist, die gegebenenfalls mit mindestens einem Halogenatom, einer Hydroxygruppe oder einer Nitrogruppe substituiert ist.

8. Verbindungen nach einem der vorhergehenden Ansprüche, dadurch gekennzeichnet, daß die Aralkylgruppen unter den Gruppen Benzyl oder Phenylethyl ausgewählt ist, die gegebenenfalls mit mindestens einem Halogenatom, einer Hydroxygruppe oder einer Nitrogruppe substituiert sind.

9. Verbindungen nach einem der vorhergehenden Ansprüche, dadurch gekennzeichnet, daß die Alkenylgruppen unter den Gruppen mit 2 bis 5 Kohlenstoffatomen, die eine oder mehrere ethylenische Doppelbindungen aufweisen, und insbesondere der Allylgruppe ausgewählt sind.

10. Verbindungen nach einem der vorhergehenden Ansprüche, dadurch gekennzeichnet, daß die Zuckerreste unter den Resten von Glucose, Galactose, Mannose oder Glucuronsäure ausgewählt sind.

11. Verbindungen nach einem der vorhergehenden Ansprüche, dadurch gekennzeichnet, daß die Aminosäurereste unter den von Lysin, Glycin oder Asparaginsäure abgeleiteten Resten ausgewählt sind.

12. Verbindungen nach einem der vorhergehenden Ansprüche, dadurch gekennzeichnet, daß die Peptidreste unter den Dipeptid- oder Tripeptidgruppen ausgewählt sind.

13. Verbindungen nach einem der vorhergehenden Ansprüche, dadurch gekennzeichnet, daß die heterocyclischen Gruppen unter den Gruppen Piperidino, Morpholino, Pyrrolidino oder Piperazino ausgewählt sind, die gegebenenfalls in 4-Stellung mit einer C₁₋₆-Alkylgruppe oder Mono- oder Polyhydroxyalkylgruppe substituiert sind.

14. Verbindungen nach einem der vorhergehenden Ansprüche, dadurch gekennzeichnet, daß die Halogenatome unter Fluor, Chlor und Brom ausgewählt sind.

15. Verbindungen nach Anspruch 1, dadurch gekennzeichnet, daß sie einzeln oder im Gemisch aus folgender Gruppe ausgewählt sind:
4-(5,6,7,8-Tetrahydro-5,5,8,8-tetramethyl-2-naphthyloxy)-benzoesäure
4-(5,6,7,8-Tetrahydro-5,5,8,8-tetramethyl-2-naphthylthio)-benzoesäure
4-(5,6,7,8-Tetrahydro-5,5,8,8-tetramethyl-2-naphthylsufinyl)-benzoesäure
4-(5,6,7,8-Tetrahydro-5,5,8,8-tetramethyl-2-naphthylsul fonyl)-benzoesäure
4-(5,6,7,8-Tetrahydro-5,5,8,8-tetramethyl-2-naphthylamino)-benzoesäure
5-(5,6,7,8-Tetrahydro-5,5,8,8-tetramethyl-2-naphthylthio)-2-thiophen-carbonsäure
4-(3,5,5,8,8-Pentamethyl-5,6,7,8-tetrahydro-2-naphthyloxy)-benzoesäure
4-(3,5,5,8,8-Pentamethyl-5,6,7,8-tetrahydro-2-naphthylthio)-benzoesäure
4-(3-Ethyl-5,5,8,8,-tetramethyl-5,6,7,8-tetrahydro-2-naphthyloxy)-benzoesäure
4-(3-Isopropyl-5,5,8,8,-tetramethyl-5,6,7,8-tetrahydro-2-naphthyloxy)-benzoesäure
4-(5,6,7,8-Tetrahydro-5,5,8,8-tetramethyl-2-naphthyloxy)-acetophenon
4-(5,6,7,8-Tetrahydro-5,5,8,8-tetramethyl-2-naphthyloxy)-benzaldehyd
4-(3-Brom-5,6,7,8-tetrahydro-5,5,8,8-tetramethyl-2-naphthyloxy)-benzoesäure
3-Methyl-4-(5,6,7,8-tetrahydro-5,5,8,8-tetramethyl-2-naphylthio)-benzoesäure
3-Methyl-4-(3,5,5,8,8-pentamethyl-5,6,7,8-tetrahydro-2-naphthylthio)-benzoesäure
3-Methyl-4-(3-ethyl-5,6,7,8-tetrahydro-5,5,8,8-tetramethyl-2-naphthyloxy)-benzoesäure
6-(3,5,5,8,8-Pentamethyl-5,6,7,8-tetrahydro-2-naphthylthio)-nicotinsäure
2-Hydroxy-4-(3,5,5,8,8-pentamethyl-5,6,7,8-tetrahydro-2-naphthylthio)-benzoesäure
2-Chlor-4-(3,5,5,8,8-pentamethyl-5,6,7,8-tetrahydro-2-naphthylthio)-benzoesäure
4-(3-Ethyl-5,6,7,8-tetrahydro-5,5,8,8-tetramethyl-2-naphthylthio)-benzoesäure
4-(3-Isopropyl-5,6,7,8-tetrahydro-5,5,8,8-tetramethyl-2-naphthylthio)-benzoesäure
4-(3-n-Propyl-5,6,7,8-tetrahydro-5,5,8,8-tetramethyl-2-naphthylthio)-benzoesäure
4-(3,5,5,8,8-Pentamethyl-5,6,7,8-tetrahydro-2-naphthythio)-benzolmethanol
4-(3,5,5,8,8-Pentamethyl-5,6,7,8-tetrahydro-2-naphthylthio)-benzaldehyd
N-Ethyl-4-(3,5,5,8,8-pentamethyl-5,6,7,8-tetrahydro-2-naphthylthio)-benzamid
N-4-Hydroxyphenyl-4-(3,5,5,8,8-pentamethyl-5,6,7,8-tetrahydro-2-naphthylthio)-benzamid

16. Verbindungen nach Anspruch 1, dadurch gekennzeichnet, daß sie mindestens eine und vorzugsweise alle folgenden Eigenschaften aufweisen: R₁ ist eine Gruppe -CO-R₇; R₂ ist eine niedere Alkylgruppe oder eine Gruppe -O-R₉; Ar ist eine Gruppe, die unter den Gruppen der Formel (a) ausgewählt ist; und X ist eine Gruppe -O-, -S- oder -NR₉.

17. Verbindungen nach einem der vorhergehenden Ansprüche für eine Verwendung als Arzneimittel.

18. Verbindungen nach Anspruch 17 für eine Verwendung als Arzneimittel, das zur Behandlung von dermatologischen Erkrankungen, rheumatischen Erkrankungen, Erkrankungen der Atemwege, kardiovaskulären Erkrankungen und ophthalmologischen Erkrankungen bestimmt ist.

19. Verwendung mindestens einer der Verbindungen nach den Ansprüchen 1 bis 16 zur Herstellung eines Arzneimittels, das zur Behandlung von dermatologischen Erkrankungen, rheumatischen Erkrankungen, Erkrankungen der Atemwege, kardiovaskulären Erkrankungen und ophthalmologischen Erkrankungen bestimmt ist.

20. Pharmazeutische Zusammensetzung, dadurch gekennzeichnet, daß sie in einem pharmazeutisch akzeptablen Träger mindestens eine der Verbindungen nach einem der Ansprüche 1 bis 16 enthält.

21. Zusammensetzung nach Anspruch 20, dadurch gekennzeichnet, daß die Konzentration der Verbindung(en) nach einem der Ansprüche 1 bis 16 im Bereich von 0,001 bis 5 Gew.-%, bezogen auf die gesamte Zusammensetzung, liegt.

22. Kosmetische Zusammensetzung, dadurch gekennzeichnet, daß sie in einem kosmetisch akzeptablen Träger mindestens eine Verbindung nach einem der Ansprüche 1 bis 16 enthält.

23. Zusammensetzung nach Anspruch 22, dadurch gekennzeichnet, daß die Konzentration der Verbindung(en) nach einem der Ansprüche 1 bis 16 im Bereich von 0,001 bis 3 Gew.-%, bezogen auf die gesamte Zusammensetzung, liegt.

24. Verwendung einer kosmetischen Zusammensetzung nach einem der Ansprüche 22 oder 23 zur Körper- oder Haarhygiene.
